(19) Europäisches Patentamt
European Patent Office
Office européen des brevets

(11) Veröffentlichungsnummer: **0 003 341**
**B1**

(12) # EUROPÄISCHE PATENTSCHRIFT

(45) Veröffentlichungstag der Patentschrift: **27.06.84**

(21) Anmeldenummer: **79100176.1**

(22) Anmeldetag: **22.01.79**

(51) Int. Cl.³: **C 07 J 5/00,** C 07 J 17/00, C 07 J 7/00, C 07 J 31/00, A 61 K 31/57 // C07J71/00

(54) **11,17-substituierte Pregnane, ihre Herstellung und Verwendung zur Herstellung von pharmazeutischen Präparaten.**

(30) Priorität: **25.01.78 DE 2803661**
**19.12.78 DE 2855465**

(43) Veröffentlichungstag der Anmeldung: **08.08.79 Patentblatt 79/16**

(45) Bekanntmachung des Hinweises auf die Patenterteilung: **27.06.84 Patentblatt 84/26**

(84) Benannte Vertragsstaaten: **BE CH DE FR GB IT LU NL SE**

(56) Entgegenhaltungen:
**GB - A - 1 152 672**
**US - A - 3 453 297**

**R.A. Scherrer and M.W. Whitehouse Antiinflammatory Agents, Vol. 1, Academic Press N.Y. 1974, S. 257, 261 and 262**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Schöttle, Ernst, Dr., Neidenburger Allee 28, D-1000 Berlin 19 (DE)**
Erfinder: **Weber, Alfred, Dr., Schützallee 56, D-1000 Berlin 37 (DE)**
Erfinder: **Kennecke, Mario, Dr., Taubertstrasse 31 f, D-1000 Berlin 33 (DE)**
Erfinder: **Dahl, Helmut, Dr., Gollanczstrasse 102, D-1000 Berlin 28 (DE)**
Erfinder: **Kapp, Joachim-Friedrich, Dr., Ludolfinger Weg 51, D-1000 Berlin 28 (DE)**
Erfinder: **Wendt, Hans, Dr., Ernst-Ring-Strasse 3, D-1000 Berlin 38 (DE)**
Erfinder: **Annen, Klaus, Dr., Seegefelder Strasse 194, D-1000 Berlin 20 (DE)**
Erfinder: **Laurent, Henry, Dr., Glambecker Weg 21, D-1000 Berlin 28 (DE)**
Erfinder: **Wiechert, Rudolf, Prof., Petzower Strasse 8 A, D-1000 Berlin 39 (DE)**

**EP 0 003 341 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue Kortikoide, Verfahren zu ihrer Herstellung, ihre Verwendung zur Herstellung von pharmazeutischen Präparaten und diese Kortikoide enthaltende pharmazeutische Präparate.

Es ist seit langem bekannt, daß man bei antiinflammatorisch wirksamen 17$\alpha$-Hydroxykortikoiden deren topische Wirksamkeit steigern kann, wenn man deren 17-Hydroxygruppe verestert. (Vergleiche hierzu das Übersichtsreferat von Thomas L. Popper und Arthur S. Watnick, »Antiinflammatory Steroids in Antiinflammatory Agents« Volume 1, Academic Press, New York, San Francisco, London (1974) Seite 268—271).

Es wurde nun gefunden, daß man die topische Wirksamkeit und/oder die Dissoziation zwischen erwünschter topischer antiinflammatorischer Wirksamkeit und unerwünschter systemischer Wirksamkeit noch steigern kann, wenn man das Wasserstoffatom der die 17$\alpha$-Hydroxygruppen dieser Kortikoide nicht durch einen Ester sondern durch einen Acetalrest oder einen Thiocetalrest substituiert.

Somit betrifft die Erfindung neue Kortikoide der allgemeinen Formel I gemäß Anspruch 1 und insbesondere die in den Ansprüchen 2 bis 75 gekennzeichneten Kortikoide.

Die neuen Kortikoide der allgemeinen Formel I gemäß Anspruch 1 können als Substituenten $R_1$ einen geradkettigen oder verzweigtkettigen 1 bis 8 oder vorzugsweise 1 bis 6 Kohlenstoffatome enthaltenden Alkylrest tragen. Solche Alkylreste sind beispielsweise der Methylrest, der Äthylrest, der Propylrest, der Isopropylrest, der Butylrest, der Isobutylrest, der tert.-Butylrest, der Pentylrest, der Isopentylrest, der Hexylrest, der Heptylrest oder der Octylrest. Der Alkylrest des Substituenten $R_1$ kann aber auch durch ein Sauerstoffatom unterbrochen sein. Solche Reste sind beispielsweise der 2-Methoxyäthoxyrest, der 3-Methoxypropyloxyrest oder der 2-Äthoxyäthoxyrest.

Als Substituenten $R_2$ können die Kortikoide der allgemeinen Formel I eine 1 bis 4 Kohlenstoffatome enthaltende Alkylgruppe, wie zum Beispiel die Methylgruppe, die Äthylgruppe, die Propylgruppe, oder die Butylgruppe tragen. Bemerkenswert sind solche Kortikoide der allgemeinen Formel I in denen $R_2$ ein Wasserstoffatom darstellt, da diese keine Diastereomerengemische bilden können.

Der Wirkungsbeginn und die Wirkungsdauer der neuen Kortikoide sowie ihre Löslichkeit in physiologisch verträglichen Lösungsmitteln sind ebenso wie bei den bekannten Kortikoiden insbesondere davon abhängig, ob und gegebenenfalls mit welcher Säure eine in der 21-Position ständige Hydroxygruppe verestert ist.

Als veresterte 21-Hydroxygruppen $R_3$ kommen Acyloxygruppen mit 1 bis 16 Kohlenstoffatomen im Acylrest in Betracht. Geeignete Acyloxygruppen sind beispielsweise solche, die sich von geradkettigen oder verzweigten, gesättigten oder ungesättigten aliphatischen Mono- oder Dicarbonsäuren ableiten, welche in üblicher Weise, beispielsweise durch Hydroxygruppen, Aminogruppen oder Halogenatome substituiert sein können.

Ferner eignen sich als Acyloxygruppen auch Reste cycloaliphatischer, aromatischer, gemischt aromatisch-aliphatischer oder heterocyclischer Säuren, die ebenfalls in üblicher Weise substituiert sein können. Als geeignete Acyloxygruppen seien beispielsweise genannt:

die Formyloxy-, Acetoxy-, Propionyloxy-, Butyryloxy-, Pentanoyloxy-, Hexanoyloxy-, Octanoyloxy-, Undecanoyloxy-, Dimethylacetoxy-, Trimethylacetoxy-, Diäthylacetoxy-, tert.-Butylacetoxy-, Benzoyloxy-, Phenacetyloxy-, Cyclopentylpropionyloxy-, Hydroxyacetoxy-, Monochloracetoxy-, Dichloracetoxy-, Trichloracetoxy-, ferner die Dimethylaminoacetoxy-, die Trimethylaminoacetoxy-, die Diäthylaminoacetoxy-, die Piperidinoacetoxy-, die Nicotinoyloxy-, die $\omega$-Carboxypropionyloxy- und die $\omega$-Carboxypentanoyloxygruppe.

Zur Herstellung wasserlöslicher Wirkstoffe können die 21-Acyloxyverbindungen mit einer basischen Stickstoffgruppe im Acylrest in die entsprechenden Säureadditionssalze, wie zum Beispiel die Hydrochloride, Hydrobromide, Sulfate, Phosphate, Oxalate, Tartrate oder Maleate überführt werden. Ferner lassen sich die 21-Dicarbonsäuremonoester zur Erhöhung der Wasserlöslichkeit in ihre Alkalisalze, wie zum Beispiel die Natrium- oder Kaliumsalze, überführen.

Die neuen Kortikoide der allgemeinen Formel I gemäß Anspruch 1 können erfindungsgemäß nach den Verfahren gemäß Anspruch 77 bis 80 hergestellt werden.

Das im Anspruch 77 gekennzeichnete Verfahren kann unter Bedingungen durchgeführt werden, die an sich bekannt sind (Synthesis 1975, 2786, J. Chem. Soc. 66), 1974, 431, J. Amer. Chem. Soc. 74, (1952), 1239, US-Patent 3 383 394 und Angew. Chemie, 90, (1978), 289).

So kann man beispielsweise die Steroide der allgemeinen Formel II mit einem Acetal der allgemeinen Formel III in Gegenwart von sauren Katalysatoren, wie zum Beispiel Perchlorsäure, p-Toluolsulfonsäure oder zweckmäßigerweise Phosphorpentoxid umsetzen. Diese Reaktion kann in Abwesenheit weiterer Lösungsmittel oder in Gegenwart von inerten Lösungsmitteln (Chloroform, Methylenchlorid, Tetrachloräthan, Tetrachlormethan, Toluol, Diäthyläther, Tetrahydrofuran und Dioxan) durchgeführt werden. Die Reaktion wird üblicherweise bei einer Reaktionstemperatur zwischen —20°C bis +50°C durchgeführt und eignet sich insbesondere zur Herstellung solcher Steroide der allgemeinen Formel I mit $R_2$ in der Bedeutung von Wasserstoff.

Andererseits kann man die Steroide der allgemeinen Formel II auch mit einem Vinyläther der allgemeinen Formel V umsetzen. Diese Reaktion wird vorzugsweise in einem der obengenannten inerten Lösungsmittel unter Zusatz saurer Katalysatoren (Perchlorsäure, p-Toluolsulfonsäure und Methansulfonsäure) durchgeführt. Vorzugsweise erfolgt die Reaktion bei einer Reaktionstemperatur von — 20° C bis 100° C.

Ferner kann man die Steroide der allgemeinen Formel II auch mit einem $\alpha$-Halogenäther der allgemeinen Formel IV umsetzen. Diese Reaktion kann beispielsweise in einem inerten polaren Lösungsmittel, wie Acetonitril, Dimethylformamid, N-Methylpyrrolidon, Hexamethylphosphorsäuretriamid oder Hexamethylphosphorsäuretriamid unter Zusatz von basischen Katalysatoren, wie z. B. Silberoxyd, Triäthylamin oder Diisopropyläthylamin durchgeführt werden. Vorzugsweise erfolgt die Reaktion bei einer Reaktionstemperatur von — 20° C bis + 100° C.

Bei den obengenannten Umsetzungen entstehen die Kortikoide der allgemeinen Formel I mit Q in der Bedeutung eines Sauerstoffatoms. Zur Herstellung von Kortikoiden mit Q in der Bedeutung eines Schwefelatoms kann man die Kortikoide der allgemeinen Formel II mit Sulfoxiden der allgemeinen Formel VI umsetzen. Diese Reaktion kann beispielsweise so durchgeführt werden, daß man das Sulfoxid und das Steroid gewünschtenfalls in einem inerten Lösungsmittel (Methylenchlorid, Tetrachloräthan und Tetrahydrofuran) unter Zusatz von Anhydriden (vorzugsweise Acetanhydrid) und Säurekatalysatoren (Essigsäure und Bortrifluorid) bei — 20° C bis 100° C umsetzt.

Verwendet man als Ausgangsverbindungen für das erfindungsgemäße Verfahren gemäß Anspruch 77 11$\beta$-Hydroxykortikoide der allgemeinen Formel II, so ist es zweckmäßig die 11$\beta$-Hydroxygruppe intermediär zu schützen, um deren teilweise Acetalisierung zu vermeiden. Dies kann beispielsweise geschehen, indem man die 11$\beta$-Hydroxygruppe vor der Acetalisierung in die entsprechenden Nitrate, Formiate oder Trihalogenacetate (insbesondere Trifluoracetate überführt und dann nach Durchführung des Verfahrens gemäß Anspruch 77 diese Ester spaltet.

Die Veresterung der 11$\beta$-Hydroxykortikoide mit Salpetersäure kann beispielsweise mit Acetylnitrat, hergestellt durch Mischen von rauchender Salpetersäure mit Essigsäureanhydrid, bewirkt werden. Nach erfolgter Acetalisierung kann man dann die Nitrate beispielsweise mittels Reaktion mit Zinkstaub in Essigsäure wieder in die 11$\beta$-Hydroxykortikoide überführen.

Die Veresterung der 11$\beta$-Hydroxykortikoide mit Ameisensäure läßt sich beispielsweise mittels Ameisensäure-Essigsäureanhydrid unter Verwendung von 4-Dimethylaminopyridin als Katalysator bewirken. Nach erfolgter 17$\alpha$-Acetalisierung kann man die erhaltenen 11$\beta$-Formyloxykortikoide dann mittels basischer Hydrolyse (beispielsweise mittels Natriummethylatlösung) oder mittels enzymatischer Verseifung in die entsprechenden 11$\beta$-Hydroxysteroide überführen.

Die Veresterung der 11$\beta$-Hydroxykortikoide mit Trihalogenessigsäure — insbesondere Trifluoressigsäure — kann beispielsweise in der Weise bewirkt werden, daß man die 11$\beta$-Hydroxykortikoide mit Trihalogenacetanhydrid in Pyridin umsetzt. Nach erfolgter 17$\alpha$-Acetalisierung kann man dann die Trihalogencacylgruppe mittels Hydrolyse (beispielsweise in einem niederen Alkohol unter Zusatz schwach basischer Katalysatoren, wie zum Beispiel Natriumacetat oder Triäthylamin) wieder abspalten.

Das im Anspruch 78 gekennzeichnete Verfahren kann ebenfalls unter Bedingungen durchgeführt werden, die an sich bekannt sind (US-A-3 678 034, 3 718 671, 3 845 085 und 3 894 063).

So kann man beispielsweise die 9,11-Dehydrosteroide der allgemeinen Formel VII in einem inerten Lösungsmittel (Essigsäure, Tetrahydrofuran, Dioxan und Acetonitril) mit Reagenzien umsetzen, die in Gegenwart von Wasser und Säuren (Schwefelsäure, Phosphorsäure und Perchlorsäure) im Verlauf der Reaktion unterchlorige Säure freisetzen, also insbesondere mit Halogenkationen bildenden Reagenzien, wie zum Beispiel N-Chloracylamide (insbesondere N-Chloracetamid) oder N-Chloracylimide (insbesondere N-Chlorsuccinimid. Bei dieser Reaktion erhält man als Hauptprodukte die 9$\alpha$-Chlor-11$\beta$-hydroxykortikoide der allgemeinen Formel I gemäß Anspruch 1, sowie häufig als Nebenprodukte die entsprechenden 9$\alpha$,11$\beta$-Dichlorkortikoide der Formel I. Letztere erhält man als Hauptprodukte, wenn man die Reaktion unter Ausschluß von Wasser in Gegenwart von Chlorwasserstoff als Säure durchführt.

Andererseits kann man den Epoxyring von 9,11-Epoxysteroiden der allgemeinen Formel VIII beispielsweise in der Weise mit Chlorwasserstoff oder Fluorwasserstoff öffnen, indem man die Verbindungen der Formel IV in einen mit Chlorwasserstoff oder Fluorwasserstoff gesättigten inerten Lösungsmittel löst und in diese Lösung gegebenenfalls noch zusätzlich Chlorwasserstoff-Gas einleitet. Geeignete inerte Lösungsmittel sind beispielsweise Äther (Diäthyläther, Diisopropyläther, Dioxan und Tetrahydrofuran) oder chlorierte Kohlenwasserstoffe (Methylenchlorid, Chloroform, Tetrachlorkohlenstoff und Tetrachloräthan). Bei dieser Umsetzung entstehen die 9$\alpha$-Fluor- bzw. 9$\alpha$-Chlor-11$\beta$-hydroxykortikoide der allgemeinen Formel I gemäß Anspruch 1.

Zur Abspaltung von Halogen aus den 9-Halogensteroiden der allgemeinen Formel IX kann man diese beispielsweise in einem inerten Lösungsmittel in Gegenwart von Radikalbildnern (Azodiisobutyronitril, Di-tert.-butylperoxyd, UV-Licht usw.) mit Trialkylzinnhydriden (Triäthylzinnhydrid und Tributylzinnhydrid) umsetzen). Geeignete inerte Lösungsmittel sind beispielsweise Äther (Diäthyläther, Glykoldimethyläther, Dioxan, Tetrahydrofuran, Kohlenwasserstoffe (Cyclohexan und Benzol, Toluol), Alkohole (Methanol, Äthanol und Isopropanol) oder Nitrile (Acetonitril). Bei dieser Umsetzung bilden

sich die in 9α-Stellung unsubstituierten 11β-Hydroxykortikoide der allgemeinen Formel I gemäß Anspruch 1.

Die Halogenwasserstoffabspaltung aus den 9-Halogensteroiden der allgemeinen Formel IX erfolgt unter den Bedingungen, welche man in der Steroidchemie üblicherweise zur Halogenwasserstoffabspaltung aus Halogenhydrinen anwendet.

So kann man beispielsweise die Verbindungen der allgemeinen Formel IX in einem tertiären Amin, wie Pyridin, Lutidin oder insbesondere Kollidin unter Rückfluß erhitzen. Eine weitere geeignete Methode zur Bromwasserstoffabspaltung ist beispielsweise die Umsetzung dieser Verbindungen mit Lithiumsalzen (Lithiumchlorid) und/oder Calziumkarbonat in Dimethylformamid oder Dimethylacetamid. Bei diesen Umsetzungen entstehen die $\Delta^8$-Kortikoide der allgemeinen Formel I gemäß Anspruch 1.

Die für das erfindungsgemäße Verfahren gemäß Anspruch 78 benötigten Ausgangssubstanzen der allgemeinen Formel VII bis IX können in der Weise hergestellt werden, indem man die entsprechenden 17α-Hydroxysteroide unter den Bedingungen des Anspruchs 77 acetalisiert oder aus den 17-acetalisierten in der 9-Positionen unsubstituierten Kortikoiden, indem man diese zu den 9,11-Dehydrosteroiden der Formel VII dehydratisiert, an die 9,11-Doppelbindung HBr anlagert, und die erhaltenen 9-Bromsteroide der Formel IX mittels Basen in die Epoxyde der Formel VIII überführt.

Die gemäß Anspruch 77a bis 77d und Anspruch 78a bis 78c erhaltenen Verfahrensprodukte können gewünschtenfalls weiter umgewandelt werden, indem die in der 1,2-Position gesättigten Kortikoide in der 1,2-Position dehydriert, und/oder eine 11β-Hydroxygruppe dieser Verbindungen zur 11-Oxogruppe oxydiert, und/oder 21-Estergruppen verseift oder 21-Hydroxygruppen verestert oder gegen Fluoratome oder Chloratome austauscht.

Die Bedingungen, unter denen die in der 1,2-Position gesättigten Kortikoide in der 1,2-Position dehydriert werden können, werden an späterer Stelle beschrieben.

Eine bevorzugte Methode besteht darin, die 21-Hydroxygruppe mit einer Sulfonsäure, vorzugsweise mit Methansulfonsäure oder p-Toluolsulfonsäure zu verestern und anschließend die Sulfonsäuregruppe gegen Halogen auszutauschen. Die Veresterung der 21-Hydroxygruppe erfolgt beispielsweise, indem man ein Sulfonsäurechlorid in Gegenwart einer organischen Base, wie Pyridin oder in Gegenwart wäßrigen Alkalis auf die 21-Hydroxysteroide einwirken läßt. Der Austausch der Sulfonsäuregruppe gegen ein Halogenatom erfolgt vorzugsweise, indem man die 21-Sulfonsäureester mit einem Alkalihalogenid, wie zum Beispiel Lithiumchlorid oder Kaliumhydrogenfluorid in Gegenwart eines polaren Lösungsmittels, wie z. B. Dimethylformamid bei einer Reaktionstemperatur von 50° C bis 180° C umsetzt.

Das erfindungsgemäße Verfahren gemäß Anspruch 79 und 80 wird unter den Bedingungen durchgeführt, die man üblicherweise zur 11β-Hydroxylierung von Steroiden mit Pilzen der Gattung Curvularia anwendet.

Zur Hydroxylierung geeignete Pilze der Gattung Curvularia sind beispielsweise Curvularia falcuta QM-102 H, Curvularia genticulata IFO (6284), Curvularia lunata NRRL 2380, NRRL 2434, ATCC 12017 oder IFO (6286) oder Curvularia maculans IFO (6292).

Es sei erwähnt, daß sich auch andere 11β-hydroxylierende Mikroorganismen als solche der Gattung Curvularia zur Durchführung des Verfahrens eignen, dies bringt in der Regel keine Vorteile gegenüber dem erfindungsgemäßen Verfahren gemäß Anspruch 79 und 80.

Unter den für diese Mikroorganismen üblicherweise verwendeten Kulturbedingungen werden in einem geeigneten Nährmedium unter Belüften, Submerskulturen angezüchtet. Dann setzt man Kulturen das Substrat (in einem geeigneten Lösungsmittel gelöst oder vorzugsweise in emulgierter Form) zu und fermentiert, bis eine maximale Substratumwandlung erreicht ist.

Geeignete Substratlösungsmittel sind beispielsweise Methanol, Äthanol, Glykolmonomethyläther, Dimethylformamid oder Dimethylsulfoxyd. Die Emulgierung des Substrates kann beispielsweise bewirkt werden, in dem man dieses in mikronisierter Form oder in einem mit Wasser mischbaren Lösungsmittel (wie Methanol, Äthanol, Aceton, Glykolmonomethyläther, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxydaddukte oder Fettsäureester von Polyglykolen. Als geeignete Emulgatoren seien die handelsüblichen Netzmittel Tegin®, Tagat®, Tween® und Span® beispielsweise genannt.

Oft ermöglicht die Emulgierung der Substrate einen erhöhten Substratdurchsatz und somit eine Steigerung der Substratkonzentration. Es ist aber selbstverständlich auch möglich, bei dem erfindungsgemäßen Verfahren andere Methoden zur Steigerung des Substratdurchsatzes, wie sie dem Fermentationsfachmann wohl bekannt sind, anzuwenden.

Die optimale Substratkonzentration, Substratzugabezeit und Fermentationsdauer ist von der Struktur des verwendeten Substrates und der Art des verwendeten Mikroorganismus abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Die sich als Gegebenenfallsmaßnahme anschließende Dehydrierung der in der 1-Position gesättigten $\Delta^4$-Steriode der allgemeinen Formel I kann sowohl mittels mikrobiologischer Arbeitsmethoden als auch mittels rein chemischer Methoden durchgeführt werden. So kann man beispielsweise die $\Delta^4$-Ste-

roide unter den üblichen Bedingungen mit Bakterienkulturen der Gattung Bacillus (zum Beispiel Bacillus lentus oder Bacillus sphaericus) oder Arthrobacter (zum Beispiel Arthrobacter simplex) in der 1-Position dehydrieren. Andererseits ist es aber auch möglich, die $\Delta^1$-Dehydrierung in der Weise durchzuführen, daß man die $\Delta^4$-Steroide mit den für diese Reaktion üblichen Oxydationsmitteln, wie zum Beispiel Selendioxyd oder 2,3-Dichlor-5,6-dicyanobenzochinon in inerten Lösungsmitteln erhitzt.

Die neuen Kortikoide der allgemeinen Formel I gemäß Anspruch 1, zeichnen sich, wie bereits erwähnt bei topischer Applikation durch eine sehr gute antiinflammatorische Wirksamkeit aus und sie besitzen eine sehr günstige Dissoziation zwischen erwünschter topischer Wirksamkeit und unerwünschter systemischer Nebenwirkung:

Die topische Wirksamkeit kann mit Hilfe des Vasokonstriktionstestes wie folgt bestimmt werden.

Der Test wird an je 8 gesunden Probanden beider Geschlechter durchgeführt, die in den letzten zwei Wochen keine lokale Corticosteroidbehandlung erhalten hatten. Nach dem Entfernen des Stratum corneum bis zum Stratum lucidum auf dem Rücken der Probanden (20—40 Tesafilm-Abrisse) werden je 0,1 g der Zubereitungen auf $cm^2$ große Felder ohne Okklusionsverband aufgetragen. Um zu vermeiden, daß die gleiche Zubereitung jeweils auf identische Hautareale appliziert wird, wird in rotierender Folge aufgetragen.

Die Vasokonstriktion wird visuell nach 4 und 8 Stunden durch den Prüfer nach folgenden Wirkungsgraden beurteilt:

1 = absolute Abblassung,
2 = geringes Resterythem,
3 = mittelgradiges Erythem, Rötungsintensität im mittleren Bereich von gestrippter, unbehandelter und nicht geschädigter Haut,
4 = Erythem mit geringen Aufhellungen,
5 = keine Ablassung oder Verstärkung des Erythems.

Die Einzelbeurteilungen werden gemittelt.

In jeder Versuchsreihe wird als Referenzsubstanz das Diflucortolon-21-valerianat (=6α,9α-Difluor-11β-hydroxy-16α-methyl-21-valeryloxy-1,4-pregnadien-3,20-dion = DFV) verwendet.

Es wird jeweils die Differenz $\Delta$ der in den einzelnen Untersuchungsreihen ermittelten mittleren Wirkungsgraden von DFV und Testsubstanz ermittelt. Positive Abweichungen $\Delta$ zeigen eine günstigere, negative Abweichungen zeigen eine ungünstigere Beurteilung der Testsubstanz im Vergleich zu DFV an.

In den nachfolgenden Tabellen sind die beobachteten Testergebnisse aufgeführt, die bei der Behandlung der Probanden mit einer 0,1 ppm Wirkstoff enthaltenden Zubereitung erzielt wird.

Die systemische Wirksamkeit der Verbindungen kann mit Hilfe des Adjuvans-Ödem-Tests wie folgt ermittelt werden:

SPF-Ratten im Gewicht von 130 bis 150 g werden zur Erzeugung eines Entzündungsherdes 0,1 ml einer 0,5%igen Mycobacterium butyricum Suspension (erhältlich von der amerikanischen Firma Difko) in die rechte Hinterpfote injiziert. Vor der Injektion mißt man das Pfotenvolumen der Ratten. 24 Stunden nach der Injektion wird das Pfotenvolumen zur Bestimmung des Ausmaßes des Ödems abermals gemessen. Anschließend appliziert man den Ratten oral oder subcutan unterschiedliche Mengen der Testsubstanz — gelöst in einem Gemisch aus 29% Benzylbenzoat und 71% Rhizinusöl. Nach weiteren 24 Stunden wird das Pfotenvolumen erneut ermittelt.

Die Kontrolltiere werden in gleicher Weise behandelt, mit dem Unterschied, daß ihnen eine testsubstanzfreie Benzylbenzoat-Rhizinusöl-Mischung injiziert wird.

Aus den erhaltenen Pfotenvolumina wird in üblicher Weise die Menge an Testsubstanz bestimmt, welche erforderlich ist, um eine 50%ige Volumenminderung des experimentell erzeugten Pfotenödems zu erzielen.

In den nachfolgenden Tabellen sind die erhaltenen Testergebnisse aufgeführt, wobei jeweils die erfindungsgemäßen Substanzen mit den strukturanalogsten, in Handelspräparaten befindlichen vorbekannten Kortikoiden verglichen werden.

Tabelle 1
Testergebnisse von Hydrocortison-Derivaten

| Nr. | Substanz | Vasokonstriktionstest Δ nach 4 h | Δ nach 8 h | Adjuvans-Ödem-Test (mg/kg Tier) $ED_{50}$ p. o. | $ED_{50}$ s. c. |
|---|---|---|---|---|---|
| 1 | 17α-Butyryloxy-11β,21-dihydroxy-4-pregnen-3,20-dion (=Hydrocortison-17-butyrat) | −0,2 | −0,3 | 54 | 13 |
| 2 | 11β,21-Dihydroxy-17α-methoxy-methoxy-4-pregnen-3,20-dion | +0,3 | +0,7 | 67 | 13 |
| 3 | 17α-Äthoxymethoxy-11β,21-di-hydroxy-4-pregnen-3,20-dion | 0,0 | +0,7 | | 14,5 |
| 4 | 11β,21-Dihydroxy-17α-propoxymethoxy-4-pregnen-3,20-dion | +0,4 | +0,4 | | ca.10 |
| 5 | 11β,21-Dihydroxy-17α-isopropoxymethoxy-4-pregnen-3,20-dion | +0,9 | +0,5 | | >30 (42%) |
| 6 | 17α-Butoxymethoxy-11β,21-di-hydroxy-4-pregnen-3,20-dion | +0,9 | +0,6 | | 20 |
| 7 | 11β,21-Dihydroxy-17α-(2'-tetra-hydropyranyloxy)-4-pregnen-4,20-dion | +0,8 | +0,6 | | 12 |

Tabelle 2
Testergebnisse von Prednisolon-Derivaten

| Nr. | Substanz | Vasokonstriktionstest Δ nach 4 h | Δ nach 8 h | Adjuvans-Ödem-Test (mg/kg Tier) $ED_{50}$ p. o. | $ED_{50}$ s. c. |
|---|---|---|---|---|---|
| 8 | 11β,17α,21-Trihydroxy-1,4-pregnadien-3,20-dion (= Prednisolon) | −0,9 | −0,8 | 8,6 | 2,6 |
| 9 | 11β,21-Dihydroxy-17α-methoxy-methoxy-1,4-pregnadien-3,20-dion | −0,2 | −0,3 | 9,8 | |
| 10 | 21-Acetoxy-11β-hydroxy-17α-methoxy-methoxy-1,4-pregnadien-3,20-dion | +0,7 | +0,4 | | >3 (25%) |
| 11 | 21-Acetoxy-11β-hydroxy-17α-methylthiomethoxy-1,4-pregnadien-3,20-dion | +0,4 | +0,4 | | ca. 3 |
| 12 | 21-Butyryloxy-11β-hydroxy-17α-methoxymethoxy-1,4-pregnadien-3,20-dion | +0,2 | +0,2 | | ca. 3 |

Tabelle 3
Testergebnisse von 9-Chlorkortikoiden

| Nr. | Substanz | Vasokonstriktionstest $\Delta$ nach 4 h | $\Delta$ nach 8 h | Adjuvans-Ödem-Test (mg/kg Tier) $ED_{50}$ p. o. | $ED_{50}$ s. c. |
|---|---|---|---|---|---|
| 13 | 9α-Chlor-11β-hydroxy-16β-methyl-17α,21-dipropionyl-oxy-1,4-pregnadien-3,20-dion (=Beclomethasen-dipropionat) | −0,3 | 0,0 | 22 | 3,0 |
| 14 | 21-Acetoxy-9α-chlor-11β-hydroxy-17α-methoxymethoxy-4-pregnen-3,20-dion | +0,4 | +0,7 | | 3,2 |
| 15 | 21-Acetoxy-9α-chlor-11β-hydroxy-17α-methoxymethoxy-1,4-pregnadien-3,20-dion | +0,9 | +1,0 | | ca. 1 |
| 16 | 21-Acetoxy-9α-chlor-11β-hydroxy-17α-(2′-methoxyäthoxymethoxy)-1,4-pregnadien-3,20-dion | +1,0 | +1,3 | ca. 8 | |

Tabelle 4
Testergebnisse von 9-Fluorkortikoiden

| Nr. | Substanz | Vasokonstriktionstest $\Delta$ nach 4 h | $\Delta$ nach 8 h | Adjuvans-Ödem-Test (mg/kg Tier) $ED_{50}$ p. o. | $ED_{50}$ s. c. |
|---|---|---|---|---|---|
| 17 | 9α-Fluor-11β-hydroxy-16β-methyl-17α,21-dipropionyloxy-1,4-pregnadien-3,20-dion (= Betamethason-dipropionat) | −0,5 | −0,7 | | 2,1 |
| 18 | 21-Chlor-9α-fluor-11β-hydroxy-16β-methyl-17α-propionyloxy-1,4-pregnadien-3,20-dion (=Clobetasol-propionat) | +0,5 | +1,1 | | 0,13 |
| 19 | 21-Chlor-9α-fluor-11β-hydroxy-17α-methoxymethoxy-1,4-pregnadien-3,20-dion | +0,5 | +0,5 | | 1,9 |
| 20 | 21-Acetoxy-9α-fluor-11β-hydroxy-17β-methoxymethoxy-4-pregnen-3,20-dion | +0,6 | +0,5 | | 2,0 |
| 21 | 9α-Fluor-11β-hydroxy-17α-methoxy-methoxy-21-propionyloxy-1,4-pregnadien-3,20-dion | +0,2 | +0,7 | | 3,0 |
| 22 | 9α-Fluor-11β-hydroxy-17α-methoxy-methoxy-16β-methoxy-21-propionyloxy-1,4-pregnadien-3,20-dion | +0,5 | +1,0 | | 3,0 |
| 23 | 21-Acetoxy-9α-fluor-11β-hydroxy-16β-methyl-17α-methylthiomethoxy-1,4-pregnadien-3,20-dion | +0,5 | +0,2 | | ca. 1 |

Tabelle 5
Testergebnisse von 6-Fluor-und-6-Methylkortikoiden

| Nr. | Substanz | Vasokonstriktionstest $\Delta$ nach 4 h | $\Delta$ nach 8 h | Adjuvans-Ödem-Test (mg/kg Tier) $ED_{50}$ p. o. | $ED_{50}$ s. c. |
|---|---|---|---|---|---|
| 24 | 6α-Fluor-11β,21-dihydroxy-16α-methyl-1,4-pregnadien-3,20-dion | −1,1 | −0,8 | | 3,5 |
| 25 | 6α-Fluor-11β,21-dihydroxy-17α-methoxymethoxy-4-pregnen-3,20-dion | +0,1 | +0,1 | | 4,0 |
| 26 | 11β,17α,21-Trihydroxy-6α-methyl-1,4-pregnadien-3,20-dion (= 6-Methylprednisolon) | −0,8 | −0,9 | | |
| 27 | 11β,21-Trihydroxy-17α-(1'-methoxyäthoxy)-6α-methyl-4-pregnen-3,20-dion | +0,6 | +0,6 | 22 | |

Die neuen Verbindungen eignen sich in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremen oder Pflaster, überführt. In den so formulierten Arzneimitteln ist die Wirkstoffkonzentration von derApplikationsform abhängig. Bei Lotionen und Salben wird vorzugsweise eine Wirkstoffkonzentration von 0,001% bis 1% verwendet.

Darüber hinaus sind die neuen Verbindungen gegebenenfalls in Kombination mit den üblichen Trägermitteln und Hilfsstoffe auch gut zur Herstellung von Inhalationsmitteln geeignet, welche zur Therapie allergischer Erkrankungen der Atemwege, wie zum Beispiel des Bronchialasthmas oder der Rhinitis verwendet werden können.

Ferner eigenen sich die neuen Kortikoide auch in Form von Kapseln, Tabletten oder Dragees, die vorzugsweise 10—200 mg Wirkstoff enthalten und oral appliziert werden oder in Form von Suspensionen, die vorzugsweise 100—500 mg Wirkstoff pro Dosiseinheit enthalten und rektal appliziert werden auch zur Behandlung allergischer Erkrankungen des Darmtraktes, wie der Kolitis ulcerosa und der Kolitis granulomatosa.

Die nachfolgenden Beispiele dienen zur Erläuterung der Erfindung.

## Beispiel 1

a) 21,63 g 3β,21-Diacetoxy-17α-hydroxy-5-pregnen-20-on werden in 150 ml wasserfreiem Methylenchlorid und 100 ml wasserfreiem Formaldehyddimethylacetal gelöst. Dann kühlt man die Lösung mit Wasser und trägt in sie ein Gemisch von 21,6 g Phosphorpentoxyd und 43 g Kieselgur ein und rührt eine Stunde lang bei Raumtemperatur. Dann filtriert man die Reaktionsmischung und wäscht den Rückstand mit Methylenchlorid, setzt dem Filtrat Triäthylamin zu bis ein pH-Wert von 9 erreicht wird und engt es im Vakuum ein. Der Rückstand wird aus Methanol-Methylenchlorid umkristallisiert und man erhält 22,68 g 3β,21-Diacetoxy-17α-methoxymethoxy-5-pregnen-20-on vom Schmelzpunkt 182—184° C.

b) Ein 2 l Erlenmeyerkolben mit 1 Liter steriler Nährlösung enthaltend 0,3% Hefeextrakt, 0,3% Cornsteep liqour und 0,2% Stärkezucker — eingestellt auf pH = 7,0 — wird mit einer Trockenkultur von Flavobacterium dehydrogenans ATCC 13 930 beimpft und bei 30° C zwei Tage lang mit 175 Umdrehungen pro Minute geschüttelt.

Ein 500 ml Erlenmeyerkolben mit 85 ml des gleichen Nährmediums wird mit 10 ml der Flavobacterium dehydrogenans Anzuchtkultur beimpft und bei 30° C 7 Stunden lang mit 175 Umdrehungen pro Minute geschüttelt. Dann setzt man der Kultur 5 ml einer sterilen Lösung von 0,5 g 3β,21-Diacetoxy-17α-methoxymethoxy-5-pregnen-20-on in Dimethylformamid zu und schüttelt bei 30° C weitere 65 Stunden lang mit 175 Umdrehungen pro Minute. Nach erfolgter Fermentation wird die Kultur zweimal mit 100 ml Äthylenchlorid extrahiert, der Extrakt im Vakuum eingeengt, der Rückstand durch Chromatographie über Aluminiumoxid gereinigt und man erhält 402 mg 21-Hydroxy-

17$\alpha$-methoxymethoxy-4-pregnen-3,20-dion vom Schmelzpunkt 152—153° C.

c) Ein 2 l Erlenmeyerkolben mit 1 Liter steriler Nährlösung enthaltend 2% Glukose und 2% Cornsteep liqour — eingestellt auf pH = 6,5 — wird mit einer Abschwemmung einer Trockenkultur von Curvularia NRRL 2380 beimpft und bei 30° C 60 Stunden lang mit 175 Umdrehungen pro Minute geschüttelt.
Ein 500 ml Erlenmeyerkolben mit 90 ml eines sterilen Nährmediums enthaltend 1,0% Cornsteep liqour und 1,25% Sojapuder — eingestellt auf pH = 6,2 — wird mit 10 ml der Curvularia lunata Anzuchtskultur beimpft und bei 30° C 7 Stunden lang mit 175 Umdrehungen pro Minute geschüttelt. Dann setzt man der Kultur 0,6 ml einer sterilen Lösung von 30 mg 21-Hydroxy-17$\alpha$-methoxymethoxy-4-pregnen-3,20-dion in Dimethylformamid zu und fermentiert weitere 65 Stunden unter den angegebenen Bedingungen.
Man arbeitet die Fermentationskultur auf, wie im Beispiel 1b beschrieben und erhält 27 mg 11$\beta$,21-Dihydroxy-17$\alpha$-methoxymethoxy-4-pregnen-3,20-dion vom Schmelzpunkt 180—182° C.

d) 2,5 g 11$\beta$,21-Dihydroxy-17$\alpha$-methoxymethoxy-4-pregnen-3,20-dion werden im 40 ml wasserfreiem Methylenchlorid gelöst, auf 0° C gekühlt und innerhalb von 15 Minuten unter Argon mit einer Lösung von 2,25 ml Titantetrachlorid in 10 ml Methylenchlorid versetzt. Man läßt die Reaktionsmischung 90 Minuten lang bei Raumtemperatur rühren, setzt dann 150 ml Methylenchlorid und 100 ml gesättigte wäßrige Natriumhydrogenkarbonatlösung zu, rührt 15 Minuten lang, trennt die organische Phase ab, wäscht sie neutral, trocknet sie über Natriumsulfat und engt sie im Vakuum ein. Der Rückstand wird aus Chloroform umkristallisiert und man erhält 2,19 g 11$\beta$,17$\alpha$,21-Trihydroxy-4-pregnen-3,20-dion vom Zersetzungspunkt 215° C.

Beispiel 2

a) 50 g 3$\beta$,21-Diacetoxy-17$\alpha$-hydroxy-5-pregnen-20-on werden mit 50 mg wasserfreier p-Toluolsulfonsäure und 350 ml wasserfreiem Methylenchlorid versetzt, auf 0° C gekühlt und nach Zugabe von 10 g Methylvinyläther 4 Stunden lang bei 0° C gerührt. Dann setzt man der Reaktion Triäthylamin zu, bis ein pH-Wert von 9 erreicht ist und engt sie im Vakuum ein. Man erhält 58 g 3$\beta$,21-Diacetoxy-17$\alpha$-(1'-methoxyäthoxy)-5-pregnen-20-on als Diastereomerengemisch vom Schmelzpunkt 80—118° C (Eine aus Methanol umkristallisierte Probe schmilzt bei 132—134° C).

b) Unter den Bedingungen des Beispiels 1b wird eine Lösung von 600 mg 3$\beta$,21-Diacetoxy-17$\alpha$-(1'-methoxyäthoxy)-5-pregnen-20-on Diastereomerengemisch in 5 ml Dimethylformamid mit einer Flavobacterium dehydrogenans ATCC 13930 Kultur umgesetzt, aufbereitet und man erhält 394 mg 21-Hydroxy-17$\alpha$-(1'methoxyäthoxy)-4-pregnen-3,20-dion als Diastereomerengemisch vom Schmelzpunkt 166—178° C.

c) Eine Lösung von 100 mg 21-Hydroxy-17$\alpha$-(1'-methoxyäthoxy)-4-pregnen-3,20-dion Diastereomerengemisch in 2 ml Dimethylformamid wird unter den im Beispiel 1c beschriebenen Bedingungen mit einer Kultur von Curvularia lunata NRRL 2380 fermentiert, aufbereitet und man erhält 105 mg 11$\beta$,21-Dihydroxy-17$\alpha$-(1'-methoxyäthoxy)-4-pregnen-3,20-dion als öliges Diastereomerengemisch.
Dieses Gemisch wird mit 3 ml Methanol und 0,5 ml 2n wäßriger Salzsäure versetzt und 5 Stunden lang bei Raumtemperatur geschüttelt. Anschließend setzt man der Mischung 4 ml Wasser zu, neutralisiert sie mit gesättigter wäßriger Natriumhydrogenkarbonat-Lösung, extrahiert zweimal mit je 8 ml Äthylenchlorid, engt die organische Phase im Vakuum ein, reinigt den Rückstand durch Chromatographie über eine Aluminiumoxid-Säule und erhält 69 mg 11$\beta$,17$\alpha$,21-Trihydroxy-4-pregnen-3,20-dion vom Schmelzpunkt 217—219° C.

Beispiel 3

a) 10,0 g 21-Acetoxy-17$\alpha$-hydroxy-4-pregnen-3,20-dion werden mit 13 mg wasserfreier p-Toluolsulfonsäure und 130 ml wasserfreiem Methylenchlorid versetzt, auf 0° C gekühlt und nach Zugabe von 2,3 g Methylvinyläther 7 Stunden lang bei 0° C gerührt. Man arbeitet die Reaktionsmischung auf, wie im Beispiel 2a beschrieben und erhält 11,6 g 21-Acetoxy-17$\alpha$-(1'methoxyäthoxy)-4-pregnen-3,20-dion vom Schmelzpunkt 135—150° C.

b) Eine Lösung von 0,1 g 21-Acetoxy-17$\alpha$-(1'-methoxyäthoxy)-4-pregnen-3,20-dion Diastereomerengemisch in 2 ml Dimethylformamid wird unter den im Beispiel 1c beschriebenen Bedingungen mit einer Kultur von Curvularia lunata NRRL 2380 fermentiert und aufbereitet. Das so erhaltene 11$\beta$,21-Dihydroxy-17$\alpha$-(1'-methoxyäthoxy)-4-pregnen-3,20-dion wird dann unter den im Beispiel 2c beschriebenen Bedingungen hydrolysiert und man erhält 78 mg 11$\beta$,17$\alpha$,21-Trihydroxy-4-pregnen-3,20-dion vom Schmelzpunkt 217—219° C.

Beispiel 4

a) 10,0 g 21-Acetoxy-17α-hydroxy-4-pregnen-3,20-dion werden unter den im Beispiel 3a beschriebenen Bedingungen mit 2,50 g Äthylvinyläther umgesetzt, aufbereitet und man erhält 12,5 g 21-Acetoxy-17α-(1'äthoxyäthoxy)-4-pregnen-3,20-dion als öliges Diastereomerengemisch.

b) Eine Lösung von 0,1 g 21-Acetoxy-17α-(1'-äthoxyäthoxy)-4-pregnen-3,20-dion Gemisch in 2 ml Dimethylformamid wird unter den im Beispiel 1c beschriebenen Bedingungen mit Curvularia lunata NRRL 2380 hydroxyliert aufbereitet und man erhält das 17α-(1'-Äthoxyäthoxy)-11β,21-dihydroxy-4-pregnen-3,20-dion, welches unter den im Beispiel 2c beschriebenen Bedingungen zu 63 mg 11β,17α,21-Trihydroxy-4-pregnen-3,20-dion vom Schmelzpunkt 216—217,5°C hydrolysiert wird.

Beispiel 5

a) 10,0 g 21-Acetoxy-17α-hydroxy-4-pregnen-3,20-dion werden unter den im Beispiel 3a beschriebenen Bedingungen mit 4,0 g Isobutylvinyläther umgesetzt, aufbereitet und man erhält 13,5 g 21-Acetoxy-17α-(1'isobutyloxyäthoxy)-4-pregnen-3,20-dion als öliges Diastereomerengemisch.

b) Eine Lösung von 0,1 g 21-Acetoxy-17α-(1'-isobutyloxyäthoxy)-4-pregnen-3,20-dion Gemisch in 2 ml Dimethylformamid wird unter den im Beispiel 1c beschriebenen Bedingungen mit Curvularia lunata NRRL 2380 hydroxyliert, aufbereitet und man erhält das 11β,21-Dihydroxy-17α-(1'-isobutyloxyäthoxy)-4-pregnen-3,20-dion welches unter den im Beispiel 2c beschriebenen Bedingungen zu 68 mg 11β,17α,21-Trihydroxy-4-pregnen-3,20-dion vom Schmelzpunkt 214—216°C (Zersetzung) verseift wird.

Beispiel 6

a) 1,95 g 21-Acetoxy-17α-hydroxy-4-pregnen-3,20-dion werden mit 5 mg wasserfreier p-Toluolsulfonsäure, 25 ml wasserfreiem Methylenchlorid und 3,5 ml Dihydropyran versetzt und 13 Stunden lang bei Raumtemperatur gerührt. Man arbeitet die Reaktionsmischung auf, wie im Beispiel 3a beschrieben und erhält 2,0 g 21-Acetoxy-17α-(2'-tetrahydropyranyloxy)-4-pregnen-3,20-dion als Diastereomerengemisch vom Schmelzpunkt 185—200°C.

b) Eine Lösung von 0,1 g 21-Acetoxy-17α-(2'-tetrahydropyranyloxyl-4-pregnen-3,20-dion Gemisch in 2 ml Dimethylformamid wird unter den Bedingungen, wie im Beispiel 1c beschrieben, mit Curvularia lunata NRRL 2380 hydroxyliert, aufbereitet und man erhält das 11β,21-Dihydroxy-17α-(2'-tetrahydropyranyloxy)-4-pregnen-3,20-dion, welches unter den im Beispiel 2c beschriebenen Bedingungen zu 72 mg 11β,17α,21-Trihydroxy-4-pregnen-3,20-dion vom Schmelzpunkt 215°C (Zersetzung) hydrolysiert wird.

Beispiel 7

a) 50 g 21-Acetoxy-17α-hydroxy-6-α-methyl-4-pregnen-3,20-dion werden unter den im Beispiel 3a beschriebenen Bedingungen mit 13,9 g Methylvinyläther umgesetzt, aufbereitet und man erhält 59 g 21-Acetoxy-17α-(1'-methoxyäthoxy)-6α-methyl-4-pregnen-3,20-dion als amorphe Masse.

b) Eine Lösung von 200 mg 21-Acetoxy-17α-(1'-methoxyäthoxy)-6α-methyl-4-pregnen-3,20-dion in 0,4 ml Dimethylformamid wird unter den im Beispiel 1c beschriebenen Bedingungen mit Curvularia Lunata NRRL 2380 hydroxyliert, aufbereitet und man erhält das 11β,21-Dihydroxy-17α-(1'-methoxyäthoxy)-6α-methyl-4-pregnen-3,20-dion, welches unter den im Beispiel 2c beschriebenen Bedingungen zu 15 mg 11β,17α,21-Trihydroxy-6α-methyl-4-pregnen-3,20-dion vom Schmelzpunkt 189—192°C hydrolysiert wird.

Beispiel 8

a) 2,0 g 21-Acetoxy-17α-hydroxy-16β-methyl-4-pregnen-3,20-dion werden mit 5 mg p-Toluolsulfonsäure (wasserfrei) und 25 ml wasserfreiem Methylenchlorid versetzt und auf 0°C gekühlt. Dann setzt man der Mischung unter Rühren 0,5 g Methylvinyläther zu, rührt 5 Stunden lang bei 0°C und weitere 12 Stunden bei Raumtemperatur, arbeitet die Reaktionsmischung auf, wie im Beispiel 3a beschrieben und erhält 2,1 g 21-Acetoxy-17α-(1'-methoxyäthoxy)-16β-methyl-4-pregnen-3,20-dion als öliges Diastereomerengemisch.

b) Eine Lösung von 50 mg 21-Acetoxy-17α-(1'-methoxyäthoxy)-16β-methyl-4-pregnen-3,20-dion Gemisch in 1 ml Dimethylformamid wird unter den im Beispiel 1c beschriebenen Bedingungen mit Curvularia lunata NRRL 2380 hydroxyliert, aufbereitet und man erhält das 11β,21-Dihydroxy-17α-(1'-methoxyäthoxy)-16β-methyl-4-pregnen-3,20-dion, welches unter den im Beispiel 2c beschriebenen Bedingungen zu 32 mg 11β,17α,21-Trihydroxy-16β-methyl-4-pregnen-3,20-dion vom

Schmelzpunkt 204—207° C hydrolysiert wird.

Beispiel 9

a) 50 g 17α-Hydroxy-4-pregnen-3,20-dion werden unter den im Beispiel 3a beschriebenen Bedingungen mit 15 g Methylvinyläther umgesetzt, aufbereitet und man erhält 51,2 g 17α-(1'-Methoxyäthoxy)-4-pregnen-3,20-dion vom Schmelzpunkt 115—152° C.

b) Eine Lösung von 0,1 g 17α-(1'-Methoxyäthoxy)-4-pregnen-3,20-dion in 1 ml Dimethylformamid wird unter den im Beispiel 1c beschriebenen Bedingungen mit einer Kultur von Curvularia lunata NRRL 2380 fermentiert und aufbereitet. Das so erhaltene 11β-Hydroxy-17α-(1'-methoxyäthoxy)-4-pregnen-3,20-dion (Schmelzpunkt 85—103° C) wird unter den im Beispiel 2c beschriebenen Bedingungen hydrolysiert und man erhält 63 mg 11β,17α-Dihydroxy-4-pregnen-3,20-dion vom Schmelzpunkt 222—223,5° C.

Beispiel 10

a) 50 g 3β,21-Diacetoxy-17α-hydroxy-5-pregnen-20-on werden unter den im Beispiel 1a beschriebenen Bedingungen in 150 ml Methylenchlorid mit 380 g Formaldehyd-bis-glykolmonomethyl-ätheracetal, 50 g Phosphorpentoxid und 100 g Kieselgur umgesetzt, aufbereitet und man erhält 45,8 g 3β,21-Diacetoxy-17α-(1,3,6-trioxaheptyl)-5-pregnen-20-on vom Schmelzpunkt 160—161° C.

b) Unter den im Beispiel 1b beschriebenen Bedingungen werden 0,5 g 3β,21-Diacetoxy-17α-(1,3,6-trioxaheptyl)-5-pregnen-20-on mit einer Kultur von Flavobacterium dehydrogenans ATCC 13930 umgesetzt, aufbereitet und man erhält 390 mg 21-Hydroxy-17α-(1,3,6-trioxaheptyl)-4-pregnen-3,20-dion als glasige Masse.

c) Unter den Bedingungen des Beispiels 1c werden 30 mg 21-Hydroxy-17α-(1,3,6-trioxaheptyl)-4-pregnen-3,20-dion mit einer Kultur von Curvularia lunata NRRL 2380 umgesetzt, aufbereitet und man erhält 24 mg 11β,21-Dihydroxy-17α-(1,3,6-trioxaheptyl)-4-pregnen-3,20-dion vom Schmelzpunkt 143—147° C.

d) Unter den Bedingungen des Beispiels 1d werden 10 mg 11β,21-Dihydroxy-17α-(1,3,6-trioxaheptyl)-4-pregnen-3,20-dion in 2 ml Methylenchlorid und 0,01 ml Titantetrachlorid umgesetzt, aufbereitet und man erhält 8 mg 11β,17α,21-Trihydroxy-4-pregnen-3,20-dion vom Zersetzungspunkt 213° C.

Beispiel 11

Ein 2 l-Erlenmeyerkolben mit 500 ml steriler Nährlösung, enthaltend

0,1% Hefeextrakt
0,5% Cornsteep liquor
0,1% Stärkezucker
— eingestellt auf pH 7,0 —

wird mit einer Abschwemmung einer Trockenkultur von Arthrobacter simplex ATCC 6946 beimpft und bei 30° C 48 Stunden lang mit 190 Umdrehungen pro Minute geschüttelt.

Ein 500 ml-Erlenmeyerkolben mit 90 ml des zuvor beschriebenen Nährmediums wird mit 10 ml der Arthrobacter simplex Anzuchtskultur beimpft und bei 30° C 6 Stunden lang mit 165 Umdrehungen pro Minute geschüttelt. Dann setzt man der Kultur 1 ml einer sterilen Lösung von 50 mg 11β,21-Dihydroxy-17α-methoxymethoxy-4-pregnen-3,20-dion in Dimethylformamid zu und fermentiert weitere 42 Stunden.

Man arbeitet die Fermentationskultur auf — wie in Beispiel 1 b beschrieben — und erhält 44,5 mg 11β,21-Dihydroxy-17α-methoxymethoxy-1,4-pregnadien-3,20-dion vom Schmelzpunkt 229/230—231° C.

Beispiel 12

Ein 2 l-Erlenmeyerkolben mit 500 ml steriler Nährlösung enthaltend

1% Hefeextrakt (Difco)
0,45% Dinatriumhydrogenphosphat
0,34% Kaliumhydrogenphosphat
0,2% Tween 80
— eingestellt auf pH 6,7 —

wird mit einer Abschwemmung einer Trockenkultur von Nocardia globerula ATCC 9356 beimpft und bei 30° C 72 Stunden lang mit 190 Umdrehungen pro Minute geschüttelt.

Ein 2 l-Erlenmeyerkolben mit 950 ml steriler Nährlösung enthaltend

2,0% Cornsteep liquor
0,3% Diammoniumhydrogenphosphat
0,25% Tween 80
— eingestellt auf pH 6,5 —

wird mit 50 ml der Nocardia globerula Anzuchtskultur beimpft und bei 30° C 24 Stunden lang mit 190 Umdrehungen pro Minute geschüttelt. Dann setzt man der Kultur 5 ml einer sterilen Lösung von 0,25 g 11β,21-Dihydroxy-17α-(1'-methoxyäthoxy)-6α-methyl-4-pregnen-3,20-dion in Dimethylformamid zu und fermentiert weitere 72 Stunden. Man arbeitet die Fermentationskultur auf — wie im Beispiel 1b beschrieben — und erhält 0,21 g 11β,21-Dihydroxy-17α-(1'-methoxyäthoxy)-6α-methyl-1,4-pregnadien-3,20-dion vom Schmelzpunkt 173° C.

## Beispiel 13

a) 50 g 21-Acetoxy-17α-hydroxy-4-pregnan-3,20-dion werden mit 600 ml Formaldehyddiäthylacetal und 600 ml Methylenchlorid suspendiert und auf —30 bis —40° C gekühlt. Unter Rühren wird eine Mischung von 75 g Phosphorpentoxid und 150 g Kieselgur eingetragen und 30 Stunden bei —30° C gerührt. Die Lösung wird filtriert und mit Triäthylamin neutralisiert. Nach dem Abdestillieren der Lösungsmittel wird nochmals mit Methanol abdestilliert und der Rückstand aus Methanol umkristallisiert. Man erhält 35,9 g 21-Acetoxy-17α-äthoxymethoxy-4-pregnen-3,20-dion, das nach nochmaliger Umkristallisation bei 137—139° C schmilzt.

b) Ein 2 l-Erlenmeyerkolben mit 1 l steriler Nährlösung enthaltend

1% Cornsteep liquor
1,25% Sojapuder
— eingestellt auf pH 6,2 —

wird mit einer Abschwemmung einer Trockenkultur von Curvularia lunata NRRL 2380 beimpft und bei 30° C 72 Stunden lang mit 175 Umdrehungen pro Minute geschüttelt.

Ein 50 l-Fermenter mit 29 l eines sterilen Nährmediums wie oben beschrieben, wird mit 1 l der Curvularia lunata-Anzuchtskultur beimpft und 24 Stunden bei 30°C unter Belüftung mit 2 $m^3$ pro Stunde gezüchtet.

Ein 50 l-Fermenter mit 36 l steriler Nährlösung wie oben beschrieben, wird mit 4 l der Curvularia lunata-Vorfermenterkultur beimpft und 10 Stunden bei 30° C unter Belüftung mit 2 $m^3$ pro Stunde und Rühren mit 220 Umdrehungen pro Minute angezüchtet. Dann setzt man der Kultur 10 g 21-Acetoxy-17α-äthoxymethoxy-4-pregnen-3,20-dion in 200 ml Äthylenglycolmonomethyläther zu. Ab der 10. Stunde wird der pH-Wert zwischen 6,5 und 7,0 gehalten. Nach weiteren 4 Stunden werden nochmals 10 g 21-Acetoxy-17α-äthoxy-methoxy-4-pregnen-3,20-dion in 200 ml Äthylenglycolmonoäthyläther zugegeben und weitere 23 Stunden unter den angegebenen Bedingungen fermentiert.

Die Fermentationskultur wird dreimal mit 10 l Äthylenchlorid extrahiert und anschließend weiter aufgearbeitet wie unter Beispiel 1b beschrieben. Man erhält 13,8 g 11β,21-Dihydroxy-17α-äthoxymethoxy-4-pregnen-3,20-dion vom Schmelzpunkt 153—154° C.

## Beispiel 14

a) 25 g 21-Acetoxy-17α-hydroxy-4-pregnen-3,20-dion werden mit 200 ml Formaldehyddipropylacetal und 320 ml Methylenchlorid suspendiert und auf —20°C gekühlt. Unter Rühren wird eine Mischung aus 49,3 g Phosphorpentoxid und 97 g Kieselgur eingetragen und 22 Stunden bei —20°C gerührt. Die Lösung wird filtriert und mit Triäthylamin neutralisiert. Das Methylenchlorid wird im Vakuum abdestilliert und die Formaldehyddipropylacetal-Phase vom abgeschiedenen Öl abgegossen. Nach dem Abdestillieren weiteren Lösungsmittels im Vakuum kristallisieren 19 g 21-Acetoxy-17α-propoxymethoxy-4-pregnen-3,20-dion vom Schmelzpunkt 145—147° C.

b) 10 g 21-Acetoxy-17α-propoxymethoxy-4-pregnen-3,20-dion werden mit 1 g Tween 80 und der dreifachen Wassermenge in der Dyno-Mühle Typ KDL (Fa. Bachofen, Basel) gemahlen. Dieses Mahlgut wird mit 1%igem $H_2O_2$ mindestens 4 Stunden sterilisiert.

Curvularia lunata NRRL 2380 wird — wie in Beispiel 13 b beschrieben — im Schüttelkolben und Vorfermenter angezüchtet und damit der Hauptfermenter beimpft. Dieser Fermenter wird ebenso angesetzt wie in Beispiel 13 b beschrieben und 10 Stunden nach den ebenfalls in Beispiel 13 b beschriebenen Bedingungen angezüchtet. Dann setzt man der Kultur das Mahlgut von 21-Acetoxy-17α-propoxymethoxy-4-pregnen-3,20-dion zu und fermentiert weitere 44 Stunden, wobei der pH-Wert zwischen 6,4 und 6,7 gehalten wird. Die Fermentationskultur wird — wie in Beispiel 13 b beschrieben — aufgearbeitet und man erhält 6,5 g 11β,21-Dihydroxy-17α-propoxymethoxy-4-pregnen-3,20-dion vom Schmelzpunkt 134/135—136° C.

Beispiel 15

a) 50 g 21-Acetoxy-17α-hydroxy-4-pregnen-3,20-dion werden mit 500 ml Formaldehyddibutylacetal und 500 ml Methylenchlorid suspendiert und auf −35° C gekühlt. Unter Rühren wird eine Mischung aus 74 g Phosphorpentoxid und 150 g Kieselgur eingetragen und 30 Stunden bei −35° C gerührt. Die Lösung wird filtriert und mit Triäthylamin neutralisiert. Das Methylenchlorid wird im Vakuum abdestilliert und die Formaldehyddibutylacetal-Phase von dem abgeschiedenen Öl abgegossen. Nach dem Abdestillieren weiteren Lösungsmittels im Vakuum kristallisieren 38,7 g 21-Acetoxy-17α-butoxymethoxy-4-pregnen-3,20-dion vom Schmelzpunkt 123,5—124,5° C.

b) 8 g 21-Acetoxy-17α-butoxymethoxy-4-pregnen-3,20-dion werden mit 0,8 g Tween 80 wie in Beispiel 14 b beschrieben, gemahlen.
Curvularia lunata NRRL 2380 wird im Schüttelkolben als Anzuchskultur, im Vorfermenter und Hauptfermenter wie in Beispiel 13 b beschrieben, gezüchtet und fermentiert. Zur 10. Stunde des Hauptfermenters wird das Substrat zugesetzt und weitere 50 Stunden fermentiert.
Die Fermentationskultur wird wie in Beispiel 13 b beschrieben, aufgearbeitet und man erhält 3,7 g 11β,21-Dihydroxy-17α-Butoxymethoxy-4-pregnen-3,20-dion vom Schmelzpunkt 79—81° C.

Beispiel 16

a) 10,60 g 21-Acetoxy-6α-fluor-17α-hydroxy-4-pregnen-3,20-dion werden in 265 ml Methylenchlorid und 47,7 ml Formaldehyddimethylacetal gelöst. Ein Gemisch aus 7,95 g Phosphorpentoxid und 15,9 g Kieselgur wird portionsweise zugegeben und die Mischung 90 Minuten unter Stickstoff bei Raumtemperatur gerührt. Die Lösung wird filtriert und mit 2,1 ml Triäthylamin versetzt. Die Lösungsmittel werden abdestilliert und der Rückstand aus Methanol umkristallisiert. Man erhält 7,6 g 21-Acetoxy-6α-fluor-17α-methoxymethoxy-4-pregnen-3,20-dion vom Schmelzpunkt 161—167° C.

b) Curvularia lunata NRRL 2380 wird — wie in Beispiel 13 b beschrieben — im Schüttelkolben, Vor- und Hauptfermenter angezüchtet. Zur 10. Stunde des Hauptfermenters werden 5 g 21-Acetoxy-6α-fluor-17α-methoxymethoxy-4-pregnen-3,20-dion in 100 ml Äthylenglycolmonomethyläther zugegeben. Der pH-Wert wird ab diesem Zeitpunkt zwischen 6,5 und 7,0 gehalten. Zur 14. Stunde werden nochmals 5 g 21-Acetoxy-6α-fluor-17α-methoxymethoxy-4-pregnen-3,20-dion in 100 ml Äthylenglycolmonomethyläther zugegeben und weitere 26 Stunden fermentiert.
Die Fermentationskultur wird — wie in Beispiel 13 b beschrieben — aufgearbeitet und man erhält 4,2 g 11β,21-Dihydroxy-6α-fluor-methoxymethoxy-4-pregnen-3,20-dion vom Schmelzpunkt 190—192° C.

Beispiel 17

a) 43 g 3β,21-Diacetoxy-17α-hydroxy-16β-methyl-5-pregnen-20-on werden in 800 ml Formaldehyddimethylacetal gelöst und auf −15° C gekühlt. In Portionen wird eine Mischung von 43 g Phosphorpentoxid und 86 g Kieselgur eingetragen und die Mischung 15 Stunden bei ca. — 15° C gerührt. Die Lösung wird filtriert, mit Triäthylamin neutralisiert und die Lösungsmittel im Vakuum abdestilliert. Der Rückstand wird mit Methanol umkristallisiert, man erhält 31,5 g 3β,21-Diacetoxy-17α-methoxymethoxy-16β-methyl-5-pregnen-20-on vom Schmelzpunkt 117—118° C.

b) Flavobacterium dehydrogenans ATCC 13930 wird wie in Beispiel 1 b), angezüchtet und fermentiert. Zur 7. Stunde setzt man der Kultur 4 ml einer sterilen Lösung von 0,2 g 3β,21-Diacetoxy-17α-methoxymethoxy-16β-methyl-5-pregnen-20-on in Dimethylformamid zu und schüttelt weitere 65 Stunden.
Nach erfolgter Fermentation wird die Kultur wie unter Beispiel 1 b) beschrieben, aufgearbeitet und man erhält 163 mg 21-Hydroxy-17α-methoxymethoxy-16β-methyl-4-pregnen-3,20-dion vom Schmelzpunkt 126/128—129° C.

c) Curvularia lunata NRRL 2380 wird — wie in Beispiel 1 c) — angezüchtet und fermentiert. Zur 7. Stunde wird der Kultur 1 ml einer sterilen Lösung von 50 mg 21-Hydroxy-17α-methoxymethoxy-16β-methyl-4-pregnen-3,20-dion in Dimethylformamid zugesetzt und weitere 65 Stunden fermentiert. Die Fermentationskultur wird — wie in Beispiel 1 b) beschrieben — aufgearbeitet und man erhält 34,5 mg 11β-21-Dihydroxy-17α-methoxymethoxy-16β-methyl-4-pregnen-3,20-dion vom Schmelzpunkt 204/205—206° C.

## Beispiel 18

3 g 11$\beta$,21-Dihydroxy-17$\alpha$-methoxymethoxy-4-pregnen-3,20-dion werden in 20 ml Pyridin mit 5 ml Essigsäureanhydrid versetzt und 5 Stunden bei Raumtemperatur gerührt. Nach Fällung in 200 ml Eiswasser wird abgesaugt und man erhält 3,23 g 21-Acetoxy-11$\beta$-hydroxy-17$\alpha$-methoxymethoxy-4-pregnen-3,20-dion, das nach Umkristallisation aus Aceton bei 172—177° C schmilzt.

## Beispiel 19

4 g 11$\beta$,21-Dihydroxy-17$\alpha$-methoxymethoxy-4-pregnen-3,20-dion werden in 25 ml Pyridin mit 8 ml Propionsäureanhydrid versetzt und 105 Minuten bei Raumtemperatur gerührt. Nach Fällung in 500 ml Eiswasser wird bis zur Spaltung des Anhydrids gerührt, abgesaugt und man erhält 4,43 g 11$\beta$-Hydroxy-17$\alpha$-methoxymethoxy-21-propionyloxy-4-pregnen-3,20-dion, das nach Umkristallisation aus Methanol bei 119—121° C schmilzt.

## Beispiel 20

4 g 11$\beta$,21-Dihydroxy-17$\alpha$-methoxymethoxy-4-pregnen-3,20-dion werden in 25 ml Pyridin mit 9 ml Buttersäureanhydrid versetzt und 105 Minuten bei Raumtemperatur gerührt. Nach Fällung in 500 ml Eiswasser wird drei Stunden nachgerührt, abgesaugt und man erhält 4,55 g 21-Butyryloxy-11$\beta$-hydroxy-17$\alpha$-methoxymethoxy-4-pregnen-3,20-dion, das nach Umkristallisation aus Methanol bei 140—142° C schmilzt.

## Beispiel 21

4 g 11$\beta$,21-Dihydroxy-17$\alpha$-methoxymethoxy-4-pregnen-3,20-dion werden in 25 ml Pyridin mit 11 ml Trimethylessigsäureanhydrid und 100 mg 4-Dimethylaminopyridin versetzt. Nach sechs Stunden bei Raumtemperatur wird Eiswasser zugefügt, mit Methylenchlorid extrahiert und dieser mit wäßriger Essigsäure, Natriumhydrogencarbonat-Lösung und Wasser gewaschen, mit Natriumsulfat getrocknet und das Lösungsmittel im Vakuum abdestilliert. Man erhält 4,8 g 11$\beta$-Hydroxy-17$\alpha$-methoxymethoxy-21-trimethylacetoxy-4-pregnen-3,20-dion, das nach Umkristallisation aus Mathanol bei 182—184° C schmilzt.

## Beispiel 22

5 g 11$\beta$,21-Dihydroxy-17$\alpha$-methoxymethoxy-1,4-pregnadien-3,20-dion werden in 30 ml Pyridin mit 8 ml Essigsäureanhydrid versetzt und 1,5 Stunden bei Raumtemperatur gerührt. Nach Fällung in 300 ml Eiswasser wird abgesaugt und man erhält 5,08 g 21-Acetoxy-11$\beta$-hydroxy-17$\alpha$-methoxymethoxy-1,4-pregnadien-3,20-dion, das nach Umkristallisation aus Methanol und etwas Methylenchlorid bei 214° C schmilzt.

## Beispiel 23

1 g 11$\beta$,21-Dihydroxy-17$\alpha$-methoxymethoxy-1,4-pregnadien-3,20-dion werden in 6 ml Pyridin mit 2 ml Buttersäureanhydrid versetzt und 1,5 Stunden bei Raumtemperatur gerührt.

Nach Fällung in Eiswasser wird zwei Stunden nachgerührt, abgesaugt und man erhält 1,11 g 21-Butyryloxy-11$\beta$-hydroxy-17$\alpha$-methoxymethoxy-1,4-pregnadien-3,20-dion, das nach Umkristallisation aus Methanol mit wenig Methylenchlorid bei 182° C schmilzt.

## Beispiel 24

0,32 g Pyridinchlorochromat werden mit 2 ml wasserfreiem Methylenchlorid versetzt und unter Rühren eine Lösung von 0,45 g 21-Acetoxy-11$\beta$-hydroxy-17$\alpha$-methoxymethoxy-4-pregnen-3,20-dion in 7 ml Methylenchlorid zugefügt. Nach 4 Stunden bei 20° C wird über Kieselgur abgesaugt, mit Methylenchlorid/Diäthyläther 1 : 1 gewaschen, mit einigen Tropfen Methanol versetzt und das Lösungsmittel im Vakuum abdestilliert. Der Rückstand wird mit Wasser verrührt und abgesaugt. Umkristallisation aus Methanol ergibt 0,28 g 21-Acetoxy-17$\alpha$-methoxymethoxy-4-pregnen-3,11,20-trion vom Schmelzpunkt 160—161° C.

Beispiel 25

6 g Cortisonacetat werden in 120 ml Formaldehyddimethylacetal und 120 ml Methylenchlorid gelöst und unter Eiskühlung mit einer Mischung von 12 g Phosphorpentoxid und 24 g Kieselgur versetzt. Nach 4,5 Stunden Rühren wird filtriert, mit Triäthylamin neutralisiert und die Lösungsmittel im Vakuum abdestilliert. Der Rückstand wird an Kieselgel mit Toluol-Essigsäureäthylester-Mischungen chromatographiert und man erhält 3,85 g 21-Acetoxy-17α-methoxymethoxy-4-pregnen-3,11,20-trion, das nach Umkristallisation aus Methanol bei 160—161° C schmilzt.

Beispiel 26

a) 2 g 21-Acetoxy-11β-hydroxy-17α-methoxymethoxy-4-pregnen-3,20-dion werden in 20 ml Pyridin gelöst und unter Eiskühlung mit 0,6 ml Thionylchlorid versetzt. Nach 30 Minuten wird in Eiswasser gefällt und abgesaugt. Man erhält 1,76 g 21-Acetoxy-17α-methoxymethoxy-4,9(11)-pregnadien-3,20-dion, das nach Umkristallisation aus Methanol mit etwas Methylenchlorid bei 194—196°C schmilzt.
b) 5,0 g 21-Acetoxy-17α-methoxymethoxy-4,9(11)-pregnadien-3,20-dion werden in 50 ml Tetrahydrofuran suspendiert und bei +20° C mit 20,56 ml 1 N Perchlorsäure und 5,14 g N-Bromsuccinimid versetzt. Es wird 15 Minuten nachgerührt. Das Reaktionsgemisch wird in eine Lösung aus 5,14 g Natriumsulfit und 350 ml Eiswasser gefällt. Das Kristallisat wird abgesaugt, mit Wasser neutral gewaschen und das noch feuchte Kristallisat wird aus Methanol/Wasser umkristallisiert. Man erhält 5,0 g 21-Acetoxy-9α-brom-11β-hydroxy-17α-methoxymethoxy-4-pregnen-3,20-dion mit einem Schmelzpunkt von 130—131° C.
c) 51,8 g 21-Acetoxy-9α-brom-11β-hydroxy-17α-methoxymethoxy-4-pregnen-3,20-dion werden in 518 ml Äthanol suspendiert und mit 45,3 g Kaliumacetat wasserfrei versetzt. Das Gemisch wird eine Stunde refluxiert und nach Abkühlen auf +20° C in 5180 ml Eis-Wasser gefällt. Das Kristallisat wird abgesaugt, mit Wasser gewaschen und bei +20° C getrocknet. Es werden 41,75 g 21-Acetoxy-9β,11β-epoxy-17α-methoxymethoxy-4-pregnen-3,20-dion erhalten, das nach Umkristallisation aus Methanol bei 138—139,5° C schmilzt.
d) 1,0 g 21-Acetoxy-9β,11β-epoxy-17α-methoxymethoxy-4-pregnen-3,20-dion wird in 10 ml Methylenchlorid gelöst und mit Eiswasser gekühlt. In langsamen Strom wird über Schwefelsäure getrocknetes Salzsäuregas eingeleitet, bis im Dünnschichtchormatogramm kein Ausgangsmaterial vorhanden ist. Das Reaktionsgemisch wird in 120 ml 1%iger Natriumbicarbonatlösung gefällt. Die Methylenchloridphase wird separiert, mit Wasser neutral gewaschen, getrocknet und zur Trockne eingeengt. Man erhält 1,1 g 21-Acetoxy-9α-chlor-11β-hydroxy-17α-methoxymethoxy-4-pregnen-3,20-dion, das nach Umkristallisation einen Schmelzpunkt von 194,5° C zeigt.

Beispiel 27

5,0 g 21-Acetoxy-17α-methoxymethoxy-4,9(11)-pregnadien-3,20 werden in 50 ml Tetrahydrofuran suspendiert und bei +20° C mit 20,56 ml 1 N Perchlorsäure und 2,78 g N-Chlorsuccinimid versetzt. Es wird 24 Stunden nachgerührt, wobei noch 3,52 ml 70% Perchlorsäure addiert werden. Das Reaktionsgemisch wird in eine Lösung aus 5,14 g Natriumsulfit und 350 ml Eiswasser gefällt. Das Kristallisat wird abgesaugt, mit Wasser neutral gewaschen, und das feuchte Kristallisat wird aus Methanol/Wasser umkristallisiert. Man erhält 1,5 g 21-Acetoxy-9α-chlor-11β-hydroxy-17α-methoxymethoxy-4-pregnen-3,20-dion, das nach Kristallisation aus Essigester und Aceton bei 189—192° C schmilzt.

Beispiel 28

10 g 9α-Fluorhydrocortisonacetat werden in 67,4 ml Methylenchlorid und 134,8 ml Methylal gelöst und mit einem Eis-Methanol-Bad gekühlt. In diese Lösung werden 10 g Phosphorpentoxid vermengt mit 20 g Kieselgur eingetragen und bei −15° C 5 Stunden nachgerührt. Die Lösung wird filtriert und mit Triäthylamin neutralisiert. Nach der Destillation des Lösungsmittels wird nochmals mit Methanol abdestilliert und der Rückstand aus Methanol kristallisiert. Durch Chromatographie an Kieselgel mit Methylenchlorid +5% Methanol werden die Produkte getrennt, und erhält 3,28 g 21-Acetoxy-9α-fluor-11β,17α-dimethoxymethoxy-4-pregnen-3,20-dion mit dem Schmelzpunkt 132—134° C, 0,56 g 21-Acetoxy-9α-fluor-11β-hydroxy-17α-methoxymethoxy-4-pregnen-3,20-dion mit dem Schmelzpunkt 211—214° C und 0,79 g 21-Acetoxy-9α-fluor-17α-hydroxy-11β-methoxymethoxy-4-pregmen-3,20-dion mit einem Schmelzpunkt von 196—199° C.

Beispiel 29

5,0 g 21-Acetoxy-9α-chlor-11β-hydroxy-17α-methoxymethoxy-4-pregnen-3,20-dion werden in 10 ml Methylenchlorid und 20 ml Methanol suspendiert und auf +3° C gekühlt. In 5 Minuten wird eine Lösung aus 0,31 g Kaliumhydroxid in 11 ml Methanol zugetropft und 80 Minuten nachgerührt. Das Reaktionsgemisch wird mit 0,34 ml Eisessig neutralisiert und in 350 ml Wasser gefällt. Das Kristallisat wird abgesaugt und getrocknet. Man erhält 2,32 g 9α-Chlor-11β,21-dihydroxy-17α-methoxymethoxy-4-pregnen-3,20-dion, das nach Umkristallisation aus Methanol/Methylenchlorid einen Schmelzpunkt von 188—189° C zeigt.

Beispiel 30

a) Eine Lösung von 5,0 g Prednisolon-21-acetat in 25 ml Pyridin wird bei —15° C tropfenweise mit 3 ml Trifluoressigsäureanhydrid versetzt und 10 Minuten bei —10° C gerührt. Man gibt auf eine Eiswasser-Kochsalzlösung und filtriert den Niederschlag ab. Den Rückstand nimmt man in Methylenchlorid auf, wäscht neutral und engt nach dem Trocknen über Natriumsulfat im Vakuum ein. Ausbeute 6,3 g 21-Acetoxy-17α-hydroxy-11β-trifluoracetoxy-1,4-pregnadien-3,20-dion.

b) 3,0 g des obigen Rohproduktes werden über Nacht in einem Gemisch aus 25 ml Dimethylsulfoxid, 15 ml Essigsäureanhydrid und 4,8 ml Eisessig bei Raumtemperatur gerührt. Die Reaktionslösung wird auf eine 10%ige Natriumcarbonat-Lösung gegeben und der Niederschlag abfiltriert. Man löst den Rückstand in Methylenchlorid und arbeitet nach dem Neutralwaschen wie üblich auf. Nach der Chromatographie an 350 mg Kieselgel mit einem Methylenchlorid-Aceton-Gradienten (0—8% Aceton) isoliert man 2,83 g 21-Acetoxy-17α-methylthio-methoxy-11β-trifluoracetoxy-1,4-pregnadien-3,20-dion.

c) 1,5 g 21-Acetoxy-17α-methylthiomethoxy-11β-trifluoracetoxy-1,4-pregnadien-3,20-dion werden in 38 ml Methanol und 1,9 ml Triäthylamin 4 Stunden bei Raumtemperatur gerührt. Man reinigt das Rohprodukt an 300 g Kieselgel mit einem Methylenchlorid-Aceton-Gradienten (0—8% Aceton) und isoliert 1,2 g 21-Acetoxy-11β-hydroxy-17α-methylthiomethoxy-1,4-pregnadien-3,20-dion, Schmelzpunkt 155° C.

Beispiel 31

a) 20,0 g 21-Acetoxy-9α-fluor-11β,17α-dihydroxy-4-pregnen-3,20-dion werden analog Beispiel 30 a) mit Trifluoressigsäureanhydrid zu 23,6 g 21-Acetoxy-9α-fluor-17α-hydroxy-11β-trifluoracetoxy-4-pregnen-3,20-dion umgesetzt.

b) 3,0 g des obigen Rohproduktes behandelt man analog Beispiel 30 b) mit Dimethylsulforid, Essigsäureanhydrid und Eisessig. Das Rohprodukt reinigt man an 300 g Kieselgel mit einem Methylenchlorid-Aceton-Graduenten (0—8% Aceton). Ausbeute 2,58 g-Acetoxy-9α-fluor-17α-methylthiomethoxy-11β-trifluoracetoxy-4-pregnen-3,20-dion in 28 ml Methanol mit 1,4 ml Triäthylamin analog Beispiel 30 c) um und reinigt das Rohprodukt an 100 g Kieselgel mit einem Methylchlorid-Aceton-Gradienten (0—12% Aceton). Ausbeute 914 mg 21-Acetoxy-9α-fluor-11β-hydroxy-17α-methylthiomethoxy-4-pregnen-3,20-dion. Schmelzpunkt 193° C.

Beispiel 32

a) Eine Suspension von 6,0 g 21-Acetoxy-17α-hydroxy-1,4,9-pregnatrien-3,20-dion in 46 ml wasserfreiem Acetonitril wird mit 11,5 ml Methoxyäthoxymethylchlorid und 11,5 ml Diisopropyläthylamin versetzt und 7,5 Stunden bei 30° C gerührt. Nach der Eiswasserfällung wird der abgesaugte Niederschlag in Methylenchlorid gelöst, neutral gewaschen und nach dem Trocknen eingeengt. Die Reinigung des Reaktionsproduktes erfolgt an 800 g Kieselgel mit einem Hexan-Essigester-Gradienten (0—30% Essigester). Ausbeute 4,9 g 21-Acetoxy-17α-(1,3,6-trioxaheptyl)-1,4,9-pregnatrien-3,20-dion. Schmelzpunkt 140° C.

b) Eine Lösung von 1,0 g 21-Acetoxy-17α-(1,3,6-trioxaheptyl)-1,4,9-pregnatrien-3,20-dion in 10 ml Dioxan wird mit 900 mg N-Chlorsuccinimid und 5 ml einer 10%igen Perchlorsäure versetzt. Man rührt 3,5 Stunden bei Raumtemperatur und gibt auf Eiswasser-Kochsalz-Natriumhydrogensulfat-Lösung. Man filtriert ab und nimmt den Rückstand in Methylenchlorid auf, wäscht neutral und engt nach dem Trocknen über Natriumsulfat ein. Das Rohprodukt wird an 100 g Kieselgel mit einem Methylenchlorid-Aceton-Gradienten (0—15% Aceton) gereinigt. Ausbeute 760 mg 21-Acetoxy-9α-chlor-11β-hydroxy-17α-(1,3,6,trioxaheptyl)-1,4-pregnadien-3,20-dion (Schmelzpunkt 204° C), sowie 180 mg 21-Acetoxy-9α-11β-dichlor-17α-(1,3,6-trioxaheptyl)-1,4-pregnadien-3,20-dion (Schmelzpunkt 148° C).

Beispiel 33

a) 4,0 g 21-Acetoxy-17$\alpha$-hydroxy-1,4,9-pregnatrien-3,20-dion werden in 28 ml wasserfreiem Methylenchlorid und 18 ml Formaldihyddimethylacetal gelöst und portionsweise mit einem Gemisch aus 6,0 g Kieselgur W 20 und 3,0 g Phosphorpentoxid versetzt. Man rührt 45 Minuten bei Raumtemperatur nach, saugt ab und eluiert den Rückstand nochmals mit Methylenchlorid, das 3—5% Triäthylamin enthält. Das Rohprodukt wird an 750 g Kieselgel mit einem Methylenchlorid-Aceton-Gradienten (0—12% Aceton) gereinigt. Ausbeute 3,3 g 21-Acetoxy-17$\alpha$-methoxymethoxy-1,4,9-pregnatrien-3,20-dion, Schmelzpunkt 160° C.

b) 1,6 g 21-Acetoxy-17$\alpha$-methoxymethoxy-1,4,9-pregnatrien-3,20-dion werden in 16 ml Dioxan gelöst und mit 1,5 g N-Chlorsuccinimid versetzt. Nach tropfenweiser Zugabe von 8 ml 10%iger wäßriger Perchlorsäure rührt man 3 Stunden bei Raumtemperatur weiter und gibt auf eine Eiswasser-Kochsalz-Natriumhydrogensulfit-Lösung. Man filtriert ab und arbeitet analog Beispiel 2 auf. Man reinigt das Rohprodukt an 175 g Kieselgel mit einem Methylenchlorid-Aceton-Gradienten (0—12% Aceton). Ausbeute 1,1 g 21-Acetoxy-9$\alpha$-chlor-11$\beta$-hydroxy-17-$\alpha$-methoxymethoxy-1,4-pregnadien-3,20-dion, (Schmelzpunkt 224° C), und 250 mg 21-Acetoxy-9$\alpha$,11$\beta$-dichlor-17$\alpha$-methoxymethoxy-1,4-pregnadien-3,20-dion, Schmelzpunkt 162° C.

Beispiel 34

a) 1,8 g 21-Acetoxy-17$\alpha$-(1,3,6-trioxaheptyl)-1,4,9-pregnatrien-3,20-dion werden in 18 ml Dioxan gelöst und mit 1,6 g N-Bromsuccinimid versetzt. Nach tropfenweiser Zugabe von 8,5 ml 10%iger wäßriger Perchlorsäure rührt man 30 Minuten bei Raumtemperatur weiter und gibt auf eine Eiswasser-Kochsalz-Natriumhydrogensulfit-Lösung. Die Aufarbeitung wird analog Beispiel 5 durchgeführt. Man erhält 2,3 g rohes 21-Acetoxy-9$\alpha$-brom-11$\beta$-hydroxy-17$\alpha$-(1,3,6-trioxaheptyl)-1,4-pregnadien-3,20-dion.

b) 2,0 g obigen Rohproduktes werden in 20 ml Hexamethylphosphorsäuretriamid gelöst und mit 2,4 g Lithiumchlorid 0,5 Stunden bei 80° C Badtemperatur gerührt. Nach der Eiswasser-Kochsalz-Fällung wird abfiltriert und wie üblich aufgearbeitet. Das Rohprodukt reinigt man an 350 g Kieselgel mit einem Methylenchlorid-Aceton-Gradienten (0—15% Aceton). Ausbeute 570 mg 21-Acetoxy-11$\beta$-hydroxy-17$\alpha$-(1,3,6-trioxaheptyl)-1,4,8-pregnatrien-3,20-dion. Schmelzpunkt 170° C.

Beispiel 35

a) 3,2 g Tristriphenylphosphinrhodium(I)-Chlorid werden in einem Gemisch aus 100 ml Methanol und 300 ml Benzol gelöst und 1,5 Stunden vorhydriert. Nach Zugabe von 4,0 g 21-Acetoxy-17$\alpha$-methoxymethoxy-1,4,9-pregnatrien-3,20-dion hydriert man 6,5 Stunden unter Normaldruck weiter. Man engt die Lösung am Rotationsverdampfer ein und reinigt den Rückstand an 400 g Kieselgel mit einem Methylenchlorid-Aceton-Gradienten (0—12% Aceton). Ausbeute 2,1 g 21-Acetoxy-17$\alpha$-methoxymethoxy-4,9-pregnadien-3,20-dion.

b) Analog Beispiel 33b) werden 1,1 g 21-Acetoxy-17$\alpha$-methoxymethoxy-4,9-pregnadien-3,20-dion mit N-Chlorsuccinimid und Perchlorsäure behandelt. Nach der Aufreinigung isoliert man 430 mg 21-Acetoxy-9$\alpha$-chlor-11$\beta$-hydroxy-17$\alpha$-methoxymethoxy-4-pregnen-3,20-dion. Schmelzpunkt 195° C.

Beispiel 36

a) 17,5 g 21-Chlor-17$\alpha$-hydroxy-4,9-pregnadien-3,20-dion werden analog Beispiel 33a) mit 236 ml Formaldihyddimethylacetal umgesetzt und aufgearbeitet. Man reinigt das Rohprodukt an 2,25 kg Kieselgel mit einem Methylenchlorid-Aceton-Gradienten (0—4% Aceton). Ausbeute 7,6 g 21-Chlor-17$\alpha$-methoxymethoxy-4,9-pregnadien-3,20-dion. Schmelzpunkt 152° C.

b) 1,8 g 21-Chlor-17$\alpha$-methoxymethoxy-4,9-pregnadien-3,20-dion werden analog Beispiel 33b) mit N-Chlorsuccinimid und Perchlorsäure behandelt. Das Rohprodukt wird an 100 g Kieselgel mit einem Methylenchlorid-Aceton-Gradienten (0—10% Aceton) gereinigt. Man isoliert 126 mg 9$\alpha$,21-Dichlor-11$\beta$-hydroxy-17$\alpha$-methoxymethoxy-4-pregnen-3,20-dion. Schmelzpunkt 197° C (Zersetzung).

Beispiel 37

a) 3,0 g 21-Fluor-17$\alpha$-hydroxy-1,4,9-pregnatrien-3,20-dion werden analog Beispiel 33a) mit 14 ml Formaldihyddimethylacetal umgesetzt. Aufgearbeitet wird unter den in Beispiel 4 beschriebenen Bedingungen. Das Rohprodukt reinigt man an 450 g Kieselgel mit dem Methylenchlorid-Aceton-Gradienten (0—8% Aceton). Ausbeute 1,5 g 21-Fluor-17α-methoxymethoxy-1,4,9-pregnatrien-3,20-dion.

b) Unter den Bedingungen des Beispiels 33b) werden 500 mg 21-Fluor-17α-methoxymethoxy-1,4,9-pregnatrien-3,20-dion mit N-Chlorsuccinimid und Perchlorsäure zur Reaktion gebracht. Nach der bereits beschriebenen Aufarbeitungsvorschrift und Reinigung an Kieselgel isoliert man 420 mg 9α-Chlor-21-fluor-11β-hydroxy-17α-methoxymethoxy-1,4-pregnadien-3,20-dion. Schmelzpunkt 245° C.

## Beispiel 38

a) 1,0 g 17α-Hydroxy-1,4,9-pregnatrien-3,20-dion werden unter den Bedingungen des Beispiels 33a) mit Formaldehyddimethylacetal umgesetzt. Man isoliert 823 mg 17α-Methoxymethoxy-1,4,9-pregnatrien-3,20-dion als Rohprodukt.

b) 823 mg obigen Rohproduktes werden analog Beispiel 33b) mit N-Chlorsuccinimid und Perchlorsäure behandelt und unter den dort beschriebenen Bedingungen aufgearbeitet und gereinigt. Ausbeute 410 mg 9α-Chlor-11β-hydroxy-17α-methoxymethoxy-1,4-pregnadien-3,20-dion. Schmelzpunkt 227° C.

## Beispiel 39

a) Analog Beispiel 34a) wird 1,0 g 21-Fluor-17α-methoxymethoxy-1,4,9-pregnatrien-3,20-dion mit 900 mg N-Bromsuccicimid und 5 ml einer 10%igen wäßrigen Perchlorsäure behandelt. Man isoliert 1,1 g 9α-Brom-fluor-11β-hydroxy-17α-methoxymethoxy-1,4-pregnadien-3,20-dion.

b) 1,1 g des rohen 9α-Brom-21-fluor-11β-hydroxy-17α-methoxymethoxy-1,4-pregnadien-3,20-dion werden mit 1,4 g Lithiumchlorid analog Beispiel 34b) zu 21-Fluor-11β-hydroxy-17α-methoxymethoxy-1,4,8-pregnatrien-3,20-dion umgesetzt. Ausbeute 490 mg. Schmelzpunkt 218° C.

## Beispiel 40

a) 3,3 g 21-Chlor-17α-hydroxy-1,4,9-pregnatrien-3,20-dion werden unter den Bedingungen des Beispiels 33a) mit Formaldihyddimethylacetal umgesetzt. Man isoliert 2,4 g 21-Chlor-17α-methoxymethoxy-1,4,9-pregnatrien-3,20-dion.

b) Man behandelt 1,4 g 21-Chlor-17α-methoxymethoxy-1,4,9-pregnatrien-3,20-dion mit N-Bromsuccinimid analog Beispiel 34a) und isoliert 1,7 g 9α-Brom-21-chlor-11β-hydroxy-17α-methoxymethoxy-1,4-pregnadien-3,20-dion als Rohprodukt.

c) Unter den Bedingungen des Beispiels 34b) setzt man 1,7 g 9α-Brom-21-chlor-11β-hydroxy-17α-methoxymethoxy-1,4-pregnadien-3,20-dion mit 2,1 g Lithiumchlorid um. Das Rohprodukt wird an 300 g Kieselgel mit einem Methylenchlorid-Aceton-Gradienten (0—8% Aceton) gereinigt. Ausbeute 530 mg 21-Chlor-11β-hydroxy-17α-methoxymethoxy-1,4,8-pregnatrien-3,20-dion. Schmelzpunkt 166° C.

## Beispiel 41

a) Man stellt analog Beispiel 38 aus 7,6 g 21-Fluor-17α-hydroxy-4,9-pregnadien-3,20-dion und 68 ml Formaldehyddimethylacetal 3,4 g 21-Fluor-17α-methoxymethoxy-4,9-pregnadien-3,20-dion her.

b) Unter den Bedingungen des Beispiels 36b) werden 1,4 g 21-Fluor-17α-methoxymethoxy-4,9-pregnadien-3,20-dion mit N-Chlorsuccinimid und Perchlorsäure behandelt. Das Rohprodukt reinigt man als 100 g Kieselgel mit einem Methylenchlorid-Aceton-Gradienten (0—10% Aceton). Ausbeute 380 mg 9α-Chlor-21-fluor-11β-hydroxy-17α-methoxymethoxy-4-pregnen-3,20-dion. Schmelzpunkt 214° C. (Zers.)

## Beispiel 42

a) In ein Gemisch aus 100 ml Pyridin und 12 ml Trifluoressigsäureanhydrid werden bei −10° C 20,0 g 9α-Fluor-11β,17α-dihydroxy-21-propionyloxy-1,4-pregnadien-3,20-dion gegeben und 10 Minuten bei −10° C nachgerührt. Nach der Eiswasserfällung filtriert man ab und nimmt den Rückstand in Methylenchlorid auf. Nach dem Waschen und Trocknen der organischen Lösung wird im Vakuum eingeengt, wo bei man 22,0 g 9α-Fluor-17α-hydroxy-21-propionyloxy-11β-trifluoracetoxy-1,4-pregnadien-3,20-dion isoliert.

b) 22,0 g des obigen Rohproduktes werden unter den Bedingungen des Beispiels 33a) mit 90 ml

Formaldehyddimethylacetal umgesetzt, wobei man 9α-Fluor-17α-methoxymethoxy-21-propionyloxy-11β-trifluoracetoxy-1,4-pregnadien-3,20-dion als Rohprodukt isoliert.

c) Man löst das rohe 9α-Fluor-17α-methoxymethoxy-21-propionyloxy-11β-trifluoracetoxy-1,4-pregnadien-3,20-dion in 500 ml Methanol und nach Zugabe von 25 ml Triäthylamin rührt man 30 Minuten bei Raumtemperatur weiter. Man engt die Reaktionslösung im Vakuum zur Trockne ein und chromatographiert den Rückstand an 2,25 kg Kieselgel mit einem Methylenchlorid-Aceton-Gradienten (0—12% Acetol). Ausbeute 12,3 g 9α-Fluor-11β-hydroxy-17α-methoxymethoxy-21-propionyloxy-1,4-pregnadien-3,20-dion. Schmelzpunkt 241° C.

Beispiel 43

a) Unter den Bedingungen des Beispiels 42a) wird 1,0 g 21-Butyryloxy-9α-fluor-11β,17-dihydroxy-1,4-pregnadien-3,20-dion mit Trifluoressigsäureanhydrid umgesetzt und aufgearbeitet. Man isoliert 0,9 g 21-Butyryloxy-9α-fluor-17α-hydroxy-11β-trifluoracetoxy-1,4-pregnadien-3,20-dion.

b) 800 mg obigen Rohproduktes werden analog Beispiel 42b) mit 3,6 ml Formaldihyddimethylacetal behandelt. Nach der Aufarbeitung erhält man 1,1 g rohes 21-Butyryloxy-9α-fluor-17α-methoxymethoxy-11β-trifluoracetoxy-1,4-pregnadien-3,20-dion.

c) 1,1 g rohes 21-Butyryloxy-9α-fluor-17α-methoxymethoxy-11β-trifluoracetoxy-1,4-pregnadien-3,20-dion werden analog Beispiel 42b) mit Triäthylamin zur Reaktion gebracht. Das Rohprodukt chromatographiert man an 75 g Kieselgel mit einem Methylenchlorid-Aceton-Gradienten (0—15% Aceton). Ausbeute 540 mg 21-Butyryloxy-9α-fluor-11β-hydroxy-17α-methoxymethoxy-1,4-pregnadien-3,20-dion. Schmelzpunkt 247° C.

Beispiel 44

2,0 g 21-Butyryloxy-9α-fluor-17α-hydroxy-1,4-pregnadien-3,11,20-trion werden analog Beispiel 33a) mit 9 ml Formaldehyddimethylacetal umgesetzt und aufgearbeitet. Man reinigt das Rohprodukt an 300 g Kieselgel mit einem Methylenchlorid-Aceton-Gradienten (0—10% Aceton). Ausbeute 2,07 g 21-Butyryloxy-9α-fluor-17α-methoxymethoxy-1,4-pregnadien-3,11,20-trion. Schmelzpunkt 192° C.

Beispiel 45

Analog Beispiel 32 werden 700 mg 21-Butyryloxy-9α-fluor-17α-hydroxy-1,4-pregnadien-3,11,20-trion mit 1,54 ml Methoxyäthoxymethylchlorid umgesetzt. Das Rohprodukt wird an 135 g Kieselgel mit einem Methylenchlorid-Aceton-Gradienten (0—5% Aceton) gereinigt. Ausbeute 430 mg 21-Butyryloxy-9α-fluor-17α-(1,3,6-trioxaheptyl)-1,4-pregnadien-3,11-20-trion. Schmelzpunkt 126° C.

Beispiel 46

a) Man behandelt 15,2 g 9α-Fluor-11β,17α-dihydroxy-16β-methyl-21-propionyloxy-1,4-pregnadien-3,20-dion, hergestellt aus 9α-Fluor-11β,17β,17α,21-trihydroxy-16β-methyl-1,4-pregnadien-3,20-dion und Propionsäureanhydrid, mit 9,1 ml Trifluoressigsäureanhydrid analog Beispiel 42a). Man erhält 15,4 g 9α-Fluor-17α-hydroxy-16β-methyl-21-propionyloxy-1β-trifluoracetoxy-1,4-pregnadien-3,20-dion.

b) 15,4 g des obigen Rohproduktes werden unter den Bedingungen des Beispiels 42b) mit Formaldehyddimethylacetal zum 9α-Fluor-17α-methoxymethoxy-16β-methyl-21-propionyloxy-11β-trifluoracetoxy-1,4-pregnadien-3,20-dion umgesetzt. Ausbeute 16,9 g des Rohproduktes.

c) Die Lösung des rohen 9α-Fluor-17α-methoxymethoxy-16β-methyl-21-propionaloxy-11β-trifluoracetoxy-1,4-pregnadien-3,20-dion in 250 ml Methanol wird analog Beispiel 42c) mit 30 ml Triäthylamin behandelt. Nach der Aufarbeitung reinigt man das Rohprodukt an 1,5 kg Kieselgel mit einem Methylenchlorid-Aceton-Gradienten (0—10% Aceton). Ausbeute 9,6 g 9α-Fluor-11β-hydroxy-17α-methoxymethoxy-16β-methyl-21-propionyloxy-1,4-pregnadien-3,20-dion. Schmelzpunkt 169° C.

Beispiel 47

600 mg 9α-Fluor-11β-hydroxy-17α-methoxymethoxy-16β-methyl-21-propionyloxy-1,4-pregnadien-3,20-dion werden mit 500 mg Tristriphenylphosphinrhodium(I)-chlorid unter den Bedingungen des Beispiels 35a) hydriert und aufgearbeitet. Nach der Chromatographie des Rohproduktes an 65 g Kieselgel mit einem Methylenchlorid-Aceton-Graduenten (0—10% Aceton) isoliert man 347 mg 9α-Fluor-11β-hydroxy-17α-methoxymethoxy-16β-methyl-21-propionyloxy-4-pregnen-3,20-dion. Schmelzpunkt 165° C.

## Beispiel 48

Eine Suspension von 6,9 g 9α-Fluor-11β-hydroxy-17α-methoxymethoxy-16β-methyl-21-propionyloxy-1,4-pregnadien-3,20-dion in 80 ml methanolischer 0,2 N Kaliumhydroxidlösung wird 45 Minuten bei 0° C gerührt. Man neutralisiert mit 10%iger Essigsäure und erhält nach der Eiswasserfällung und Aufarbeitung ein Rohprodukt, das 450 g Kieselgel mit einem Methylenchlorid-Aceton-Graduenten (0—20% Aceton) gereinigt wird. Ausbeute 4,1 g 9α-Fluor-11β,21-dihydroxy-17α-methoxymethoxy-16β-methyl-1,4-pregnadien-3,20-dion. Schmelzpunkt 220° C.

## Beispiel 49

a) Eine Lösung von 1,7 g 9α-Fluor-11β,21-dihydroxy-17α-methoxymethoxy-16β-methyl-1,4-pregnadien-3,20-dion in 17 ml Pyridin wird mit 2,04 g Tosylchlorid 1 Stunde bei Raumtemperatur gerührt. Nach der Eiswasserfällung wird der Niederschlag in Methylenchlorid aufgenommen und wie üblich aufgearbeitet. Man reinigt das Rohprodukt an 135 g Kieselgel mit einem Methylenchlorid-Aceton-Gradienten (0—10% Aceton). Ausbeute 876 mg 9α-Fluor-11β-hydroxy-17α-methoxymethoxy-16β-methyl-21-tosyloxy-1,4-pregnadien-3,20-dion.

b) 876 mg obigen Produktes werden in 17 ml Hexamethylphosphorsäuretriamid mit 880 mg Lithiumchlorid 1 Stunde bei 80° C gerührt. Man gibt auf Eiswasser und filtriert den Niederschlag ab. Nach der üblichen Aufarbeitung kristallisiert man das Rohprodukt aus Hexan/Aceton um. Ausbeute 485 mg 21-Chlor-9α-fluor-11β-hydroxy-17α-methoxymethoxy-16β-methyl-pregnadien-3,20-dion. Schmelzpunkt 204° C.

## Beispiel 50

a) Eine Suspension von 11,2 g 9α-Fluor-11β-hydroxy-17α-methoxamethoxy-21-propionyloxy-1,4-pregnadien-3,20-dion wird mit 129 ml einer methanolischen 0,2 N Kaliumhydroridlösung versetzt. Man rührt 1 Stunde bei Raumtemperatur und arbeitet auf, wie in Beispiel 48 beschrieben. Nach der Chromatographie an 1,5 kg Kieselgel mit einem Methylenchlorid-Aceton-Grad enden (0—35% Aceton) isoliert man 7,5 g 9α-Fluor-11β,21-dihydroxy-17α-methoxymethoxy-1,4-pregnadien-3,20-dion.

b) Analog Beispiel 49a wird 1,0 g obigen Rohprodukts mit 2,0 g Tosylchlorid umgesetzt. Das Rohprodukt reinigt man an 200 g Kieselgel mit einem Methylenchlorid-Aceton-Graduenten (0—10% Aceton). Ausbeute 886 mg 9α-Fluor-11β-hydroxy-17α-methoxymethoxy-21-toxyloxy-1,4-pregnadien-3,20-dion.

c) 886 mg 9α-Fluor-11β-hydroxy-17α-methoxymethoxy-21-toxyloxy-1,4-pregnadien-3,20-dion werden unter den Bedingungen des Beispiels 49b) mit Lithiumchlorid behandelt und aufgearbeitet. Die Reinigung erfolgt durch Umkristallisation aus Aceton/Hexan. Ausbeute 392 mg 21-Chlor-9α-fluor-11β-hydroxy-17α-methoxymethoxy-1,4-pregnadien-3,20-dion. Schmelzpunkt 225° C.

## Beispiel 51

a) Man setzt 20,0 g 21-Acetoxy-9α-fluor-11β,17-dihydroxy-4-pregnen-3,20-dion analog Beispiel 42a) mit 12 ml Trifluoremysäureanhydrid zum 21-Acetoxy-9α-fluor-17α-hydroxy-11β-trifluoracetoxy-4-pregnen-3,20-dion um.

b) 5,0 g obigen Rohproduktes werden analog Beispiel 33a) mit 22,5 ml Formaldihyddimethylacetal in das 21-Acetoxy-9α-fluor-17α-methoxymethoxy-11β-trifluoracetoxy-4-pregnen-3,20-dion umgewandelt. Ausbeute 5,3 g.

c) 5,3 g 21-Acetoxy-9α-fluor-17α-methoxymethoxy-11β-trifluoracetoxy-4-pregnen-3,20-dion werden analog Beispiel 42c) mit Triäthylamin behandelt. Man reinigt das Rohprodukt an 500 g Kieselgel mit einem Methylenchlorid-Aceton-Gradienten (0—8% Aceton). Ausbeute 560 mg 21-Acetoxy-9α-fluor-11β-hydroxy-17α-methoxymethoxy-4-pregnen-3,20-dion. Schmelzpunkt 213° C.

## Beispiel 52

1,0 g 21-Butyryloxy-9α-fluor-11β-hydroxy-17β-methoxymethoxy-1,4-pregnadien-3,20-dion werden mit 800 mg Tristriphenylphosphinrhodium(I)-chlorid unter den Bedingungen des Beispiels 35a) hydriert und aufgearbeitet. Nach der Chromatographie des Rohproduktes an 100 g Kieselgel mit einem

Methylenchlorid-Aceton-Gradienten (0—10% Aceton) isoliert man 620 mg 21-Butyryloxy-9α-fluor-11β-hydroxy-17α-methoxymethoxy-4-pregnen-3,20-dion. Schmelzpunkt 183° C.

Beispiel 53

a) Analog Beispiel 48 werden 28,0 g 21-Acetoxy-9α-fluor-11β-hydroxy-17α-methoxymethoxy-4-pregnen-3,20-dion mit methanolischer 0,2 N Kaliumhydroxidlösung zu 9α-Fluor-11β,21-dihydroxy-17α-methoxymethoxy-4-pregnen-3,20-dion verseift.

b) Eine Lösung von 500 mg 9α-Fluor-11β,21-dihydroxy-17α-methoxymethoxy-4-pregnen-3,20-dion in 5 ml Pyridin wird mit 7,5 ml n-Valeriansäureanhydrid 1 Stunde bei Raumtemperatur gerührt. Nach Eiswasserfällung filtriert man ab und arbeitet wie üblich auf. Das Rohprodukt wird an 400 g Kieselgel mit einem Hexan-Essigester-Gradienten (0—30% Essigester) gereinigt. Ausbeute 235 mg 9α-Fluor-11β-hydroxy-17α-methoxymethoxy-21-valeryloxy-4-pregnen-3,20-dion. Schmelzpunkt 181° C.

Beispiel 54

a) 29,1 g 21-Acetoxy-6-17α-hydroxy-4,6-pregnadien-3,20-dion werden in 730 ml Methylenchlorid und 131,0 ml Formaldehyddimethylacetal gelöst. Ein Gemisch aus 22,12 g Phosphorpentoxid und 44 g Kieselgur wird portionsweise zugegeben und die Mischung 2,5 Stunden unter Stickstoff bei Raumtemperatur gerührt. Die Lösung wird filtriert und mit 5,8 ml Triäthylamin versetzt. Die Lösungsmittel werden abdestilliert und der Rückstand aus Methanol unter Zusatz von Aktivkohle und 1% Triäthylamin umkristallisiert. Man erhält 15,6 g 21-Acetoxy-6-chlor-17α-methoxymethoxy-4,6-pregnadien-3,20-dion vom Schmelzpunkt 183—186° C.

b) Curvularia lunata NRRL 2380 wird — wie in Beispiel 13b beschrieben — im Schüttelkolben, Vor- und Hauptfermenter angezüchtet. Zur 10. Stunde des Hauptfermenters werden 3 g 21-Acetoxy-6-chlor-17α-methoxymethoxy-4,6-pregnadien-3,20-dion in 60 ml Äthylenglycolmonomethyläther zugegeben. Der pH-Wert wird ab diesem Zeitpunkt zwischen 6,4 und 6,7 gehalten und weitere 20 Stunden fermentiert. Die Fermentationskultur wird — wie in Beispiel 13b beschrieben — aufgearbeitet und man erhält 1,8 g 11β,21-Dihydroxy-6-chlor-17α-methoxymethoxy-4,6-pregnadien-3,20-dion vom Schmelzpunkt 234°/235—236° C.

Beispiel 55

a) 38,85 g 21-Acetoxy-17α-hydroxy-4-pregnen-3,20-dion werden mit 235 ml Formaldehyddiisopropylacetal und 500 ml Methylenchlorid verrührt und auf −20° C gekühlt. Unter Führen wird eine Mischung aus 75 g Phosphorpentoxid und 150 g Kieselgur eingetragen und 20 Stunden bei −20° C gerührt. Die Mischung wird filtriert, mit Methylenchlorid gewaschen und mit Triäthylamin auf pH 9 gebracht. Die Lösungsmittel werden im Vakuum abdestilliert und der Rückstand in Methylenchlorid aufgenommen. Die Lösung wird mit halbgesättigter Natriumchloridlösung gewaschen, mit Natriumsulfat getrocknet, mit Aktivkohle behandelt, über Kieselgur abgesaugt und im Vakuum konzentriert. Der Rückstand wird an Kieselgel mit Toluol-Essigester-Gemischen chromatographiert. Man erhält 35,8 g 21-Acetoxy-17α-isopropoxymethoxy-4-pregnen-3,20-dion, das nach Kristallisation mit Pentan einen Schmelzpunkt von 111—117° C zeigt.

b) Curvularia lunata NRRL 2380 wird — wie im Beispiel 13b beschrieben — im Schüttelkolben, Vor- und Hauptfermenter angezüchtet. Zur 10. Stunde des Hauptfermenters werden 12 g 21-Acetoxy-17α-isopropoxymethoxy-4-pregnen-3,20-dion in 240 ml Äthylenglycolmonomethyläther zugegeben. Der pH-Wert wird ab diesem Zeitpunkt zwischen 6,5 und 7,0 gehalten und weitere 15 Stunden fermentiert. Die Fermentationskultur wird — wie im Beispiel 13b beschrieben — aufgearbeitet und man erhält 8,4 g 11β,21-Dihydroxy-17α-isopropoxymethoxy-4-pregnen-3,20-dion vom Schmelzpunkt 71°/73—78° C.

c) Arthrobacter simplex ATCC 6946 wird — wie in Beispiel 11 beschrieben — im Anzuchts- und Fermentationskolben angezüchtet. Zur 6. Stunde wird dem Fermentationskolben 1 ml einer sterilen Lösung von 50 mg 11β,21-Dihydroxy-17α-isopropoxymethoxy-4-pregnen-3,20-dion in Äthylenglycolmonomethyläther zugegeben und weitere 42 Stunden fermentiert.
Die Fermentationskultur wird — wie in Beispiel 1b beschrieben — aufgearbeitet und man erhält 32 mg 11β,21-Dihydroxy-17α-isopropoxymethoxy-1,4-pregnadien-3,20-dion vom Schmelzpunkt 58°/63—65° C.

Beispiel 56

a) 10,0 g Prednisolon-21-acetat werden mit 40 g 4-Dimethylaminopyridin und 500 ml Methylenchlorid gelöst, auf −15° C gekühlt und mit 25 ml Essigsäureanhydrid versetzt. Innerhalb von 10 Minuten werden 10 ml Ameisensäure zugetropft und weitere 135 Minuten bei −10 bis −15° C gerührt. Die

Lösung wird mit Wasser, 4prozentiger Salzsäure und Natriumhydrogencarbonatlösung extrahiert, die organische Phase mit Natriumsulfat getrocknet und im Vakuum konzentriert. Der Rückstand wird aus Methanol unter Zusatz von etwas Methylenchlorid umkristallisiert und man erhält 9,34 g 21-Acetoxy-11β-formyloxy-17α-hydroxy-1,4-pregnadien-3,20-dion vom Schmelzpunkt 221—223°C.

b) 5,0 g 21-Acetoxy-11β-formyloxy-17α-hydroxy-1,4-pregnadien-3,20-dion werden in 150 ml Methylenchlorid und 30 ml Formaldehyddiäthylacetal gelöst und auf 0°C gekühlt. Unter Rühren wird eine Mischung aus 5 g Phosphorpentoxid und 10 g Kieselgur eingetragen und 2,5 Stunden im Eisbad gerührt. Die Mischung wird filtriert, mit Methylenchlorid gewaschen und mit Triäthylamin auf pH 9 gebracht. Nach dem Abdestillieren der Lösungsmittel werden 10,5 g rohes 21-Acetoxy-17α-äthoxymethoxy-11β-formyloxy-1,4-pregnadien-3,20-dion als halbfeste Masse erhalten.

Beispiel 56

c) 0,34 g rohes 21-Acetoxy-17α-äthoxymethoxy-11β-formyloxy-1,4-pregnadien-3,20-dion werden in 13 ml Methynol gelöst und unter Argon zu einer Lösung von 0,126 g Natriumhydrogencarbonat in 1,32 ml Wasser von 60°C gegeben. Die Mischung wird 10 Minuten unter Rückfluß erhitzt, abgekühlt, mit Wasser versetzt und mit Methylenchlorid extrahiert. Die Methylenchloridlösung wird mit Natriumsulfat getrocknet, konzentriert und der Rückstand über Dieselgel mit Toluol-Essigester-Gemischen chromatographiert. Man erhält 0,1 g 17α-Äthoxymethoxy-11β,21-dihydroxy-1,4-pregnadien-3,20-dion vom Schmelzpunkt 149,5—152°C.

Beispiel 57

a) 20,0 g 11β,21-Dihydroxy-17α-methoxymethoxy-4-pregnen-3,20-dion werden in 200 ml Pyridin gelöst und unter Kühlung 20 ml Methansulfonsäurechlorid zugetropft. Es wird 30 Minuten bei Raumtemperatur gerührt und in 2000 ml Eiswasser gefällt. Man erhält nach dem Absaugen, Waschen und Trocknen 23,85 g 11β-Hydroxy-21-methansulfonyloxy-17α-methoxymethoxy-4-pregnen-3,20-dion vom Zersetzungspunkt 154—155°C.

b) 20 g 11β-Hydroxy-21-methansulfonyloxy-17α-methoxymethoxy-4-pregnen-3,20-dion werden mit 600 ml Aceton versetzt, eine Lösung von 20 g Natriumjodid in 520 ml Aceton zugefügt und 11 Stunden unter Rückfluß erhitzt. Nach dem Abkühlen wird vom Unlöslichen abfiltriert, die Acetonlösung im Vakuum konzentriert und der Rückstand mit Wasser und etwas Natriumthiosulfatlösung versetzt. Das Rohprodukt wird abgesaugt, mit Wasser gewaschen und aus Aceton umkristallisiert. Man erhält 16,6 g 11β-Hydroxy-21-jod-17α-methoxymethoxy-4-pregnen-3,20-dion vom Zersetzungspunkt 123—126°C.

c) 1 g 11β-Hydroxy-21-jod-17α-methoxymethoxy-4-pregnen-3,20-dion werden mit 20 ml Toluol und 1,3 ml Tributylzinnhydrid unter Argon 30 Minuten auf 55°C erwärmt. Danach wird das Toluol im Vakuum abdestilliert und der Rückstand mit Pentan behandelt. Als Kristallisat werden 0,64 g 11β-Hydroxy-17α-methoxymethoxy-4-pregnen-3,20-dion vom Schmelzpunkt 191—194°C erhalten.

Beispiel 58

a) 9,0 g 21-Acetoxy-17α-hydroxy-4,9(11)-pregnadien-3,20-dion werden mit 72 ml Formaldehyddiäthylacetal und 280 ml Methylenchlorid gelöst und auf 0°C gekühlt. Unter Rühren wird eine Mischung aus 9,0 g Phosphorpentoxid und 18 g Kieselgur eingetragen und 2,5 Stunden im Eisbad gerührt. Die Mischung wird filtriert und mit Methylenchlorid gewaschen. Die Lösung wird mit Triäthylamin auf pH 9 gebracht, konzentriert und der Rückstand an Kieselgel mit Toluol-Essigester-Gemischen chromatographiert. Man erhält 5,93 g 21-Acetoxy-17α-äthoxymethoxy-4,9(11)-pregnadien-3,20-dion vom Schmelzpunkt 167—169°C.

b) 5,8 g 21-Acetoxy-17α-äthoxymethoxy-4,9(11)-pregnadien-3,20-dion werden unter den im Beispiel 26b genannten Bedingungen, aber ohne Umkristallisation, umgesetzt und man erhält 7,6 g rohes 21-Acetoxy-17α-äthoxymethoxy-9α-brom-11β-hydroxy-4-pregnen-3,20-dion als glasartige Substanz.

c) 7,6 g rohes 21-Acetoxy-17α-äthoxymethoxy-9α-brom-11β-hydroxy-5-pregnen-3,20-dion werden unter den im Beispiel 26c genannten Bedingungen umgesetzt und die methanolische Lösung des Rohproduktes über Kieselgel filtriert. Man erhält 5,49 g rohes 21-Acetoxy-17α-äthoxymethoxy-9β-11β-epoxy-4-pregnen-3,20-dion als amorphe Substanz.

d) 5,4 g rohes 21-Acetoxy-17α-äthoxymethoxy-9β,11β-epoxy-4-pregnen-3,20-dion werden unter den im Beispiel 26d genannten Bedingungen umgesetzt. Das Rohprodukt wird an Kieselgel mit Toluol-Essigester-Gemischen chromatographiert und man erhält 1,78 g 21-Acetoxy-17α-äthoxymethoxy-9α-chlor-11β-hydroxy-4-pregnen-3,20-dion vom Schmelzpunkt 148—151°C.

Beispiel 59

a) 6,0 g Hydrocortison-21-acetat-11-formiat werden in 150 ml Methylenchlorid und 63 g Formaldehyddihexylacetal gelöst und auf 10° C gekühlt. Unter Argon wird ein Gemisch aus 6 g Phorphorpentoxid und 12 g Kieselgur portionsweise zugegeben und die Mischung eine Stunde bei 15° C gerührt. Die Mischung wird filtriert, mit Methylenchlorid gewaschen und mit Triäthylamin auf pH 8 gebracht. Das Methylenchlorid wird im Vakuum verdampft und der Rückstand im Eisbad gekühlt. Die Lösung wird vom ausgefallenen Öl dekantiert und an Kieselgel mit Toluol-Essigester-Gemischen chromatographiert. Man erhält 4,2 g 21-Acetoxy-11$\beta$-formyloxy-17$\alpha$-hexyloxymethoxy-4-pregnen-3,20-dion, das nach Kristallisation mit Pentan bei 110° C schmilzt.

b) 0,38 g 21-Acetoxy-11$\beta$-formyloxy-17$\alpha$-hexyloxymethoxy-4-pregnen-3,20-dion werden unter den im Beispiel 56c beschriebenen Bedingungen umgesetzt und man erhält 0,1 g 11$\beta$,21-Dihydroxy-17$\alpha$-hexyloxymethoxy-4-pregnen-3,20-dion.

Beispiel 60

a) 6,0 g Hydrocortison-21-acetat-11-formiat werden in 150 ml Methylenchlorid und 65 g Formaldehyddibenzylacetal gelöst und im Wasserbad von Raumtemperatur unter Argon ein Gemisch aus 6 g Phophorpentoxid und 12 g Kieselgur portionsweise unter Rühren zugegeben. Nach vier Stunden wird die Mischung filtriert, mit Methylenchlorid gewaschen und mit Triäthylamin auf pH 8 gebracht. Das Methylenchlorid wird im Vakuum verdampft und der Rückstand an Kieselgel mit Toluol-Essigester-Mischungen chromatographiert. Man erhält 3,0 g 21-Acetoxy-17$\alpha$-benzyloxymethoxy-11$\beta$-formyloxy-4-pregnen-3,20-dion.

b) 0,38 g 21-Acetoxy-17$\alpha$-benzyloxymethoxy-11$\beta$-formyloxy-4-pregnen-3,20-dion werden unter den im Beispiel 56c beschriebenen Bedingungen umgesetzt und man erhält 0,11 g 17$\alpha$-Benzyloxymethoxy-11$\beta$,21-dihydroxy-4-pregnen-3,20-dion.

Beispiel 61

a) Flavobacterium dehydrogenans ATCC 13930 wird — wie in Beispiel 56c beschrieben — im Schüttelkolben, Vor- und Hauptfermenter abgezüchtet. Zur 24. Stunde des Hauptfermenters werden 19,5 g 16-Methylen-3$\beta$,21-diacetoxy-17$\alpha$-methoxymethoxy-5-pregnen-20-on in 500 ml Dimethylformamid zugegeben und weitere 28 Std. fermentiert.
Die Fermentationskultur wird — wie im Beispiel 13b beschrieben — aufgearbeitet und man erhält 15,5 g 16-Methylen-21-hydroxy-17$\alpha$-methoxymethoxy-4-pregnen-3,20-dion vom Schmelzpunkt 147°/150—151° C.

b) Curvularia lunata NRRL 2380 wird — wie im Beispiel 13b beschrieben — im Schüttelkolben, Vor- und Hauptfermenter angezüchtet. Zur 10. Std. des Hauptfermenters werden 20 g 16-Methylen-21-hydroxy-17$\alpha$-methoxymethoxy-4-pregnen-3,20-dion in 400 ml Äthylenglycolmonomethyläther zugegeben. Der pH-Wert wird ab diesem Zeitpunkt zwischen 6,4 und 6,7 gehalten und weitere 8 Std. fermentiert.
Die Fermentationskultur wird — wie in Beispiel 13b beschrieben — aufgearbeitet und man erhält 11 g 16-Methylen-11$\beta$,21-dihydroxy-17$\alpha$-methoxymethoxy-4-pregnen-3,20-dion vom Schmelzpunkt 205°/206—208° C.

Beispiel 62

Arthrobacter simplex ATCC 6946 wird — wie in Beispiel 11 beschrieben — in Anzuchts- und Fermentationskolben angezüchtet. Zur 6. Stunde wird dem Fermentationskolben 1 ml einer sterilen Lösung von 50 mg 11$\beta$,21-Dihydroxy-17$\alpha$-propoxymethoxy-4-pregnen-3,20-dion in Äthylenglycolmonomethyläther zugegeben und weitere 42 Stunden fermentiert. Die Fermentationskultur wird — wie in Beispiel 1b geschrieben — aufgearbeitet und man erhält 41 mg 11$\beta$,21-Dihydroxy-17$\alpha$-propoxymethoxy-1,4-pregnadien-3,20-dion vom Schmelzpunkt 121°/125—127° C.

Beispiel 63

3,0 g 11$\beta$,21-Dihydroxy-17$\alpha$-methoxymethoxy-16-methylen-4-pregnen-3,20-dion werden in 12 ml Pyridin bei +20° C gelöst und mit 2,37 ml Essigsäureanhydrid versetzt. Das Reaktionsgemisch wird 2 Stunden bei +20° C nachgerührt und anschließend in 144 ml Eis-Wasser gefällt. Es wird eine Stunde nachgerührt. Das Kristallisat wird abgesaugt, mit Wasser gewaschen und getrocknet. Nach Umkristallisation aus Essigester werden 2,74 g 21-Acetoxy-11$\beta$-hydroxy-17$\alpha$-methoxymethoxy-16-methylen-4-pregnen-3,20-dion mit einem Schmelzpunkt von 166—167° C erhalten.

## Patentansprüche

1. Kortikoide der allgemeinen Formel I

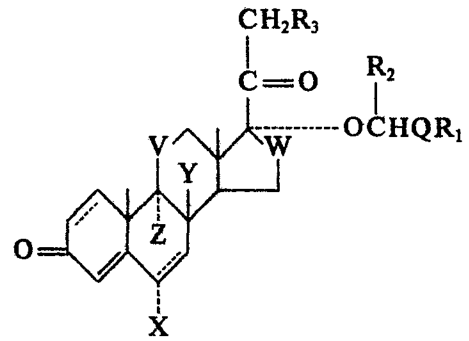

(I)

worin
die Bindungen $=$ Einfachbindungen oder Doppelbindungen,
X ein Wasserstoffatom, ein Fluoratom, ein Chloratom oder eine Methylgruppe,
Y ein Wasserstoffatom und
Z ein Wasserstoffatom, ein Fluoratom oder ein Chloratom oder
Y und Z gemeinsam eine Kohlenstoff-Kohlenstoffbindung,
V eine $\beta$-Hydroxymethylengruppe, eine $\beta$-Chlormethylengruppe oder eine Carbonylgruppe,
W eine Methylengruppe, eine Äthylidengruppe oder eine Vinylidengruppe,
Q ein Sauerstoffatom oder ein Schwefelatom,
$R_1$ eine 1 bis 8 Kohlenstoffatome enthaltende, gegebenenfalls durch ein Sauerstoffatom unterbrochene Alkylgruppe oder eine Benzylgruppe und
$R_2$ ein Wasserstoffatom oder eine 1 bis 4 Kohlenstoffatome enthaltende Alkylgruppe oder
$R_1$ und $R_2$ gemeinsam eine Trimethylengruppe oder eine Tetramethylengruppe und
$R_3$ ein Wasserstoffatom, ein Fluoratom, ein Chloratom, eine Hydroxygruppe oder eine Acyloxygruppe mit 1 bis 16 Kohlenstoffatomen bedeuten.

2. Kortikoide der allgemeinen Formel Ia gemäß Anspruch 1

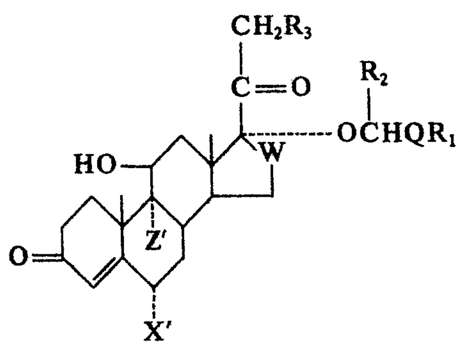

(Ia)

worin
W, Q, $R_1$, $R_2$ und $R_3$ die im Anspruch 1 genannte Bedeutung besitzen und
X′ ein Wasserstoffatom, ein Fluoratom oder eine Methylgruppe und
Z′ ein Wasserstoffatom, ein Fluoratom oder ein Chloratom bedeuten.

3. Kortikoide der allgemeinen Formel Ib gemäß Anspruch 1

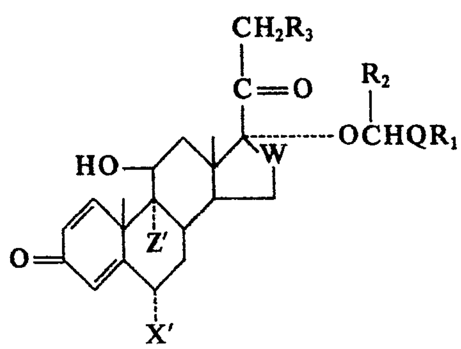

(Ib)

worin
X', Z', W, Q, $R_1$, $R_2$ und $R_3$ die im Anspruch 2 genannte Bedeutung besitzen.

4. Kortikoide der allgemeinen Formel Ic gemäß Anspruch 1

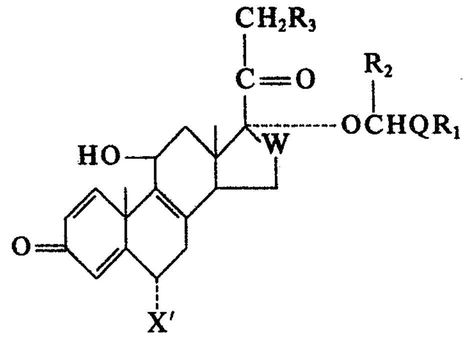

(Ic)

worin
X', W, Q, $R_1$, $R_2$ und $R_3$ die im Anspruch 2 genannte Bedeutung besitzen.

5. Kortikoide gemäß Anspruch 2 bis 4, dadurch gekennzeichnet, daß das Symbol X' der allgemeinen Formel ein Wasserstoffatom bedeutet.

6. Kortikoide gemäß Anspruch 2, 3 und 5, dadurch gekennzeichnet, daß das Symbol Z' ihrer allgemeinen Formel ein Wasserstoffatom bedeutet.

7. Kortikoide gemäß Anspruch 2, 3 und 5, dadurch gekennzeichnet, daß das Symbol Z' ihrer allgemeinen Formel ein Fluoratom oder ein Chloratom bedeutet.

8. Kortikoide gemäß Anspruch 1 bis 7, dadurch gekennzeichnet, daß das Symbol W ihrer allgemeinen Formel eine Methylengruppe bedeutet.

9. Kortikoide gemäß Anspruch 1 bis 8, dadurch gekennzeichnet, daß das Symbol Q ihrer allgemeinen Formel ein Sauerstoffatom bedeutet.

10. Kortikoide gemäß Anspruch 1 bis 8, dadurch gekennzeichnet, daß das Symbol Q ihrer allgemeinen Formel ein Schwefelatom bedeutet.

11. Kortikoide gemäß Anspruch 1 bis 10, dadurch gekennzeichnet, daß das Symbol $R_2$ ihrer allgemeinen Formel ein Wasserstoffatom bedeutet.

12. Kortikoide gemäß Anspruch 1 bis 11, dadurch gekennzeichnet, daß das Symbol $R_2$ ihrer allgemeinen Formel eine 1 bis 6 Kohlenstoffatome enthaltende Alkylgruppe bedeutet.

13. Kortikoide gemäß Anspruch 1 bis 12, dadurch gekennzeichnet, daß das Symbol $R_3$ ihrer allgemeinen Formel eine Hydroxygruppe oder eine 1 bis 8 Kohlenstoffatome enthaltende Acyloxygruppe bedeutet.

14. Kortikoide gemäß Anspruch 1 bis 12, dadurch gekennzeichnet, daß das Symbol $R_3$ ihrer allgemeinen Formel ein Fluoratom oder ein Chloratom bedeutet.

15. Verbindung gemäß Anspruch 1, nämlich 11β,21-Dihydroxy-17α-methoxymethoxy-4-pregnen-3,20-dion.

16. Verbindung gemäß Anspruch 1, nämlich 11β,21-Dihydroxy-17α-(1'-methoxyethoxy)-4-pregnen-3,20-dion.

17. Verbindung gemäß Anspruch 1, nämlich 17α-(1'-Ethoxy-ethoxy)-11β,21-dihydroxy-4-pregnen-3,20-dion.

18. Verbindung gemäß Anspruch 1, nämlich 11β,21-Dihydroxy-17α-(1'-isobutyloxy-ethoxy)-4-pregnen-3,20-dion.

19. Verbindung gemäß Anspruch 1, nämlich 11β,21-Dihydroxy-17α-(2'-tetrahydropyranyloxy)-4-pregnen-3,20-dion.

20. Verbindung gemäß Anspruch 1, nämlich 11β,21-Dihydroxy-17α-(1'-methoxyethoxy)-6α-methyl-4-pregnen-3,20-dion.

21. Verbindung gemäß Anspruch 1, nämlich 11β,21-Dihydroxy-17α-(1'-methoxyethoxy)-16β-methyl-4-pregnen-3,20-dion.

22. Verbindung gemäß Anspruch 1, nämlich 11β-Hydroxy-17α-(1'-methoxyethoxy)-4-pregnen-3,20-dion.

23. Verbindung gemäß Anspruch 1, nämlich 11β,21-Dihydroxy-17α-(2'-methoxyethoxymethoxy)-4-pregnen-3,20-dion.

24. Verbindung gemäß Anspruch 1, nämlich 11β,21-Dihydroxy-17α-methoxymethoxy-1,4-pregnadien-3,20-dion.

25. Verbindung gemäß Anspruch 1, nämlich 11β,21-Dihydroxy-17α-(1'-methoxyethoxy)-6α-methyl-1,4-pregnadien-3,20-dion.

26. Verbindung gemäß Anspruch 1, nämlich 11β,21-Dihydroxy-17α-ethoxymethoxy-4-pregnen-3,20-dion.

27. Verbindung gemäß Anspruch 1, nämlich 11β,21-Dihydroxy-17α-propoxymethoxy-4-pregnen-3,20-dion.

28. Verbindung gemäß Anspruch 1, nämlich 11β,21-Dihydroxy-17α-butoxymethoxy-4-pregnen-3,20-dion.

29. Verbindung gemäß Anspruch 1, nämlich 11β,21-Dihydroxy-6α-fluor-17α-methoxymethoxy-4-pregnen-3,20-dion.

30. Verbindung gemäß Anspruch 1, nämlich 11β,21-Dihydroxy-17α-methoxymethoxy-16β-methyl-4-pregnen-3,20-dion.

31. Verbindung gemäß Anspruch 1, nämlich 21-Acetoxy-11β-hydroxy-17α-methoxymethoxy-4-pregnen-3,20-dion.

32. Verbindung gemäß Anspruch 1, nämlich 11β-Hydroxy-17α-methoxymethoxy-21-propionyloxy-4-pregnen-3,20-dion.

33. Verbindung gemäß Anspruch 1, nämlich 21-Butyryloxy-11β-hydroxy-17α-methoxymethoxy-4-pregnen-3,20-dion.

34. Verbindung gemäß Anspruch 1, nämlich 11β-Hydroxy-17α-methoxymethoxy-21-trimethylacetoxy-4-pregnen-3,20-dion.

35. Verbindung gemäß Anspruch 1, nämlich 21-Acetoxy-11β-hydroxy-17α-methoxymethoxy-1,4-pregnadien-3,20-dion.

36. Verbindung gemäß Anspruch 1, nämlich 21-Butyryloxy-11β-17α-methoxymethoxy-1,4-pregnadien-3,20-dion.

37. Verbindung gemäß Anspruch 1, nämlich 21-Acetoxy-17α-methoxymethoxy-4-pregnen-3,11,20-trion.

38. Verbindung gemäß Anspruch 1, nämlich 21-Acetoxy-9α-chlor-11β-hydroxy-17α-methoxymethoxy-4-pregnen-3,20-dion.

39. Verbindung gemäß Anspruch 1, nämlich 21-Acetoxy-9α-fluor-11β-hydroxy-17α-methoxymethoxy-4-pregnen-3,20-dion.

40. Verbindung gemäß Anspruch 1, nämlich 9α-Chlor-11β,21-dihydroxy-17α-methoxymethoxy-4-pregnen-3,20-dion.

41. Verbindung gemäß Anspruch 1, nämlich 21-Acetoxy-11β-hydroxy-17α-methylthiomethoxy-1,4-pregnadien-3,20-dion.

42. Verbindung gemäß Anspruch 1, nämlich 21-Acetoxy-9α-fluor-11β-hydroxy-17α-methylthiomethoxy-4-pregnen-3,20-dion.

43. Verbindung gemäß Anspruch 1, nämlich 21-Acetoxy-9α-chlor-17α-(1,3,6-trioxaheptyl)-1,4-pregnadien-3,20-dion.

44. Verbindung gemäß Anspruch 1, nämlich 21-Acetoxy-9α,11β-dichlor-17α-(1,3,6-trioxaheptyl)-1,4-pregnadien-3,20-dion.

45. Verbindung gemäß Anspruch 1, nämlich 21-Acetoxy-9α-chlor-11β-hydroxy-17α-methoxymethoxy-1,4-pregnadien-3,20-dion.

46. Verbindung gemäß Anspruch 1, nämlich 21-Acetoxy-9α,11β-dichlor-17α-methoxymethoxy-1,4-pregnadien-3,20-dion.

47. Verbindung gemäß Anspruch 1, nämlich 21-Acetoxy-11β-hydroxy-17α-(1,3,6-trioxaheptyl)-1,4,8-pregnatrien-3,20-dion.

48. Verbindung gemäß Anspruch 1, nämlich 9α,21-Dichlor-11β-hydroxy-17α-methoxymethoxy-4-pregnen-3,20-dion.

49. Verbindung gemäß Anspruch 1, nämlich 9α-Chlor-21-fluor-11β-hydroxy-17α-methoxymethoxy-1,4-pregnadien-3,20-dion.

50. Verbindung gemäß Anspruch 1, nämlich 9α-Chlor-11β-hydroxy-17α-methoxymethoxy-1,4-pregnadien-3,20-dion.

51. Verbindung gemäß Anspruch 1, nämlich 21-Fluor-11β-hydroxy-17α-methoxymethoxy-1,4,8-pregnatrien-3,20-dion.

52. Verbindung gemäß Anspruch 1, nämlich 21-Chlor-11β-hydroxy-17α-methoxymethoxy-1,4,8-pregnatrien-3,20-dion.

53. Verbindung gemäß Anspruch 1, nämlich 9α-Chlor-21-fluor-11β-hydroxy-17α-methoxymethoxy-4-pregnen-3,20-dion.

54. Verbindung gemäß Anspruch 1, nämlich 9α-Fluor-11β-hydroxy-17α-methoxymethoxy-21-propionyloxy-1,4-pregnadien-3,20-dion.

55. Verbindung gemäß Anspruch 1, nämlich 21-Butyryloxy-9α-fluor-11β-hydroxy-17α-methoxymethoxy-1,4-pregnadien-3,20-dion.

56. Verbindung gemäß Anspruch 1, nämlich 21-Butyryloxy-9α-fluor-17α-methoxymethoxy-1,4-pregnadien-3,11,20-trion.

57. Verbindung gemäß Anspruch 1, nämlich 21-Butyryloxy-9α-fluor-17α-(1,3,6-trioxaheptyl)-1,4-pregnadien-3,11,20-trion.

58. Verbindung gemäß Anspruch 1, nämlich 9α-Fluor-11β-hydroxy-17α-methoxymethoxy-16β-methyl-21-propionyloxy-1,4-pregnadien-3,20-dion.

59. Verbindung gemäß Anspruch 1, nämlich 9α-Fluor-11β-hydroxy-17α-methoxymethoxy-16β-methyl-21-propionyloxy-4-pregnen-3,20-dion.

60. Verbindung gemäß Anspruch 1, nämlich 9α-Fluor-11β,21-dihydroxy-17α-methoxymethoxy-16β-methyl-1,4-pregnadien-3,20-dion.

61. Verbindung gemäß Anspruch 1, nämlich 21-Chlor-9α-fluor-11β-hydroxy-17α-methoxymethoxy-16β-methyl-1,4-pregnadien-3,20-dion.

62. Verbindung gemäß Anspruch 1, nämlich 21-Butyryloxy-9α-fluor-11β-hydroxy-17α-methoxymethoxy-4-pregnen-3,20-dion.

63. Verbindung gemäß Anspruch 1, nämlich 9α-Fluor-11β-hydroxy-17α-methoxymethoxy-21-valeryloxy-4-pregnen-3,20-dion.

64. Verbindung gemäß Anspruch 1, nämlich 21-Chlor-9α-fluor-11β-hydroxy-17α-methoxymethoxy-1,4-pregnadien-3,20-dion.

65. Verbindung gemäß Anspruch 1, nämlich 11β,21-Dihydroxy-6-chlor-17α-methoxymethoxy-4,6-pregnadien-3,20-dion.

66. Verbindung gemäß Anspruch 1, nämlich 11β,21-Dihydroxy-17α-isopropoxymethoxy-4-pregnen-3,20-dion.

67. Verbindung gemäß Anspruch 1, nämlich 11β,21-Dihydroxy-17α-isopropoxymethoxy-1,4-pregnadien-3,20-dion.

68. Verbindung gemäß Anspruch 1, nämlich 17α-Ethoxymethoxy-11β,21-dihydroxy-1,4-pregnadien-3,20-dion.

69. Verbindung gemäß Anspruch 1, nämlich 11β-Hydroxy-17α-methoxymethoxy-4-pregnen-3,20-dion.

70. Verbindung gemäß Anspruch 1, nämlich 21-Acetoxy-17α-ethoxymethoxy-9α-chlor-11β-hydroxy-4-pregnen-3,20-dion.

71. Verbindung gemäß Anspruch 1, nämlich 11β,21-Dihydroxy-17α-hexyloxymethoxy-4-pregnen-3,20-dion.

72. Verbindung gemäß Anspruch 1, nämlich 17α-Benzyloxymethoxy-11β,21-dihydroxy-4-pregnen-3,20-dion.

73. Verbindung gemäß Anspruch 1, nämlich 11β,21-Dihydroxy-17α-methoxymethoxy-16-methylen-4-pregnen-3,20-dion.

74. Verbindung gemäß Anspruch 1, nämlich 11β,21-Dihydroxy-17α-propoxymethoxy-1,4-pregnadien-3,20-dion.

75. Verbindung gemäß Anspruch 1, nämlich 21-Acetoxy-11β-hydroxy-17α-methoxymethoxy-16-methylen-4-pregnen-3,20-dion.

76. Pharmazeutische Präparate, gekennzeichnet durch einen Gehalt von 1 bis 3 Kortikoiden gemäß Anspruch 1 bis 75.

77. Verfahren zur Herstellung von Kortikoiden der allgemeinen Formel I gemäß Anspruch 1, dadurch gekennzeichnet, daß man in an sich bekannter Weise ein 17α-Hydroxysteroid der allgemeinen Formel II

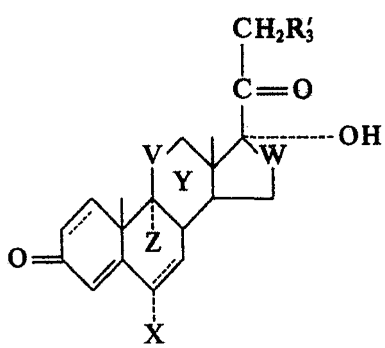

(II)

worin

, X,Y,Z,V,W die im Anspruch 1 genannte Bedeutung besitzen und $R_3'$ ein Wasserstoffatom, ein Fluoratom, ein Chloratom oder eine veresterte Hydroxygruppe bedeutet, gewünschtenfalls nach intermediarem Schutz einer 11β-Hydroxygruppe,

a) mit einem Acetal der allgemeinen Formel III

$R_2HC(OR_1)_2$ (III)

worin
$R_1$ und $R_2$ die im Anspruch 1 genannte Bedeutung besitzen umsetzt, oder

b) mit einem α-Halogenäther der allgemeinen Formel IV

$Hal-R_2CH-OR_1$ (IV)

worin
$R_1$ und $R_2$ die im Anspruch 1 genannte Bedeutung besitzen und Hal ein Chloratom ein Bromatom oder ein Jodatom darstellt, umsetzt, oder

c) mit einem Vinyläther der allgemeinen Formel V

$R_2'CH=CH-OR_1$ (V)

worin
$R_1$ die im Anspruch 1 genannte Bedeutung besitzt und $R_2'$ ein Wasserstoffatom oder einen ein bis drei Kohlenstoffatome enthaltender Alkylrest darstellt, umsetzt, oder

d) mit einem Sulfoxid der allgemeinen Formel VI

$R_2CH_2SOR_1$ (VI)

worin
$R_1$ und $R_2$ die im Anspruch 1 genannte Bedeutung besitzen umsetzt, und gegebenenfalls die gemäß Variante a bis d erhaltenen, in der 1 und 2 Position gesättigten Kortikoide in der 1,2-Position dehydriert, und/oder eine 11β-Hydroxygruppe zur Oxogruppe oxydiert, und/oder 21-Estergruppen verseift oder 21-Hydroxygruppen verestert oder gegen Fluoratome oder Chloratome austauscht.

78. Verfahren zur Herstellung von Kortikoiden der allgemeinen Formel I gemäß Anspruch 1, dadurch gekennzeichnet, daß man in an sich bekannter Weise

a) an ein 9,11-Dehydrosteroid der allgemeinen Formel VII

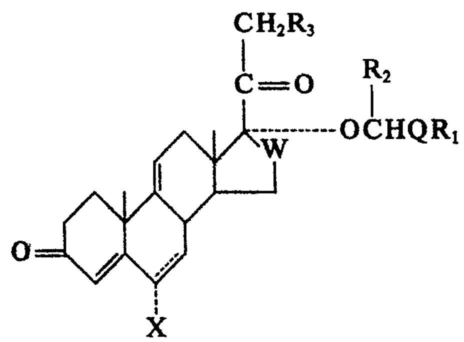

(VII)

worin
$\overline{\underline{\cdots}}$ , X, W, Q, $R_1$, $R_2$ und $R_3$ die im Anspruch 1 genannte Bedeutung besitzen, Chlor oder unterchlorige Säure anlagert, oder

b) den Epoxyring eines 9,11-Epoxysteroides der allgemeinen Formel VIII

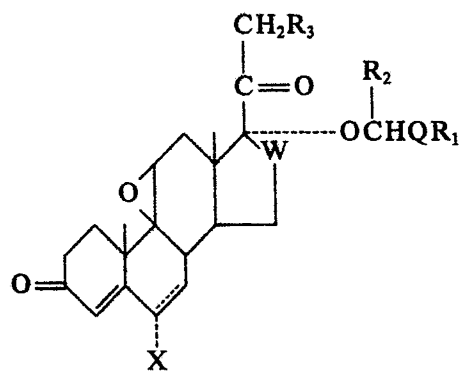

(VIII)

worin
$\overline{\underline{\cdots}}$ , X, W, Q, $R_1$, $R_2$ und $R_3$ die im Anspruch 1 genannte Bedeutung besitzen mit Fluorwasserstoff oder Chlorwasserstoff öffnet, oder

c) aus einem 9-Halogensteroid der allgemeinen Formel IX

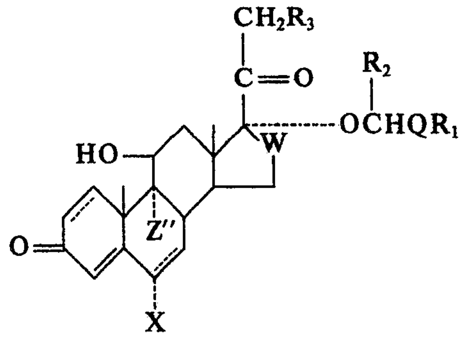

(IX)

worin
==== , X, W, Q, $R_1$, $R_2$ und $R_3$ die im Anspruch 1 genannte Bedeutung besitzen und Z″ ein Chloratom oder ein Bromatom darstellt,
Z″ oder HZ″ abspaltet,
und gegebenenfalls die gemäß Variante a bis c erhaltenen, in der 1,2-Position gesättigten Kortikoide in der 1,2-Position dehydriert, und/oder eine 11$\beta$-Hydroxygruppe zur Oxogruppe oxydiert, und/oder 21-Estergruppen verseift oder 21-Hydroxygruppen verestert oder gegen Fluoratome oder Chloratome austauscht.

79. Verfahren zur Herstellung von 11$\beta$-Hydroxysteroiden der allgemeinen Formel Id

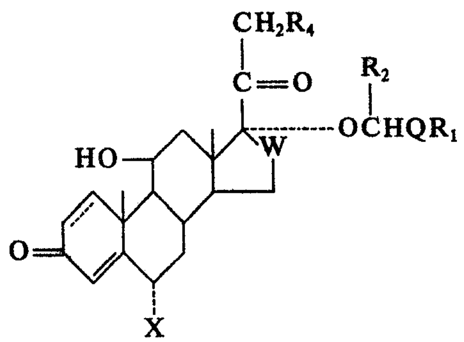

(Id)

worin
==== , X, W, Q, $R_1$ und $R_2$ die im Anspruch 1 genannte Bedeutung besitzen und $R_4$ ein Wasserstoffatom oder eine Hydroxygruppe darstellt, dadurch gekennzeichnet, daß man ein 11-Desoxysteroid der allgemeinen Formel XI

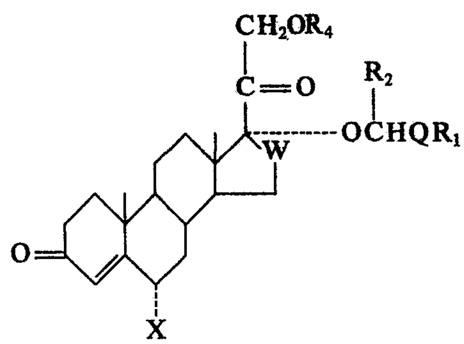

(XI)

worin
X, W, Q, $R_1$ und $R_2$ die obengenannte Bedeutung besitzen und $R_4$ ein Wasserstoffatom, eine Hydroxygruppe oder eine 1 bis 6 Kohlenstoffatome enthaltende Alkanoyloxygruppe darstellt,
mit Pilzkulturen der Gattung Curvularia fermentiert, und die erhaltenen Verbindungen der Formel Id mit ==== in der Bedeutung einer Einfachbindung gegebenenfalls in der 1,2-Position dehydriert.

80. Verfahren gemäß Anspruch 79, dadurch gekennzeichnet, daß man die Fermentation mit Hilfe von Pilzkulturen der Spezies Curvularia lunata durchführt.

**Claims**

1. Corticoids of the general formula I

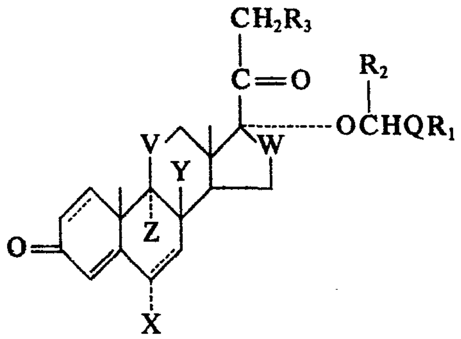

(I)

in which
the bonds ⩶ represent single bonds or double bonds,
X represents a hydrogen atom, a fluorine atom, a chlorine atom or a methyl group,
Y represents a hydrogen atom, and
Z represents a hydrogen atom, a fluorine atom or a chlorine atom, or
Y and Z together represent a carbon-carbon bond,
V represents a β-hydroxymethylene group, a β-chloromethylene group or a carbonyl group,
W represents a methylene group, an ethylidene group or a vinylidene group,
Q represents an oxygen atom or a sulphur atom,
$R_1$ represents an alkyl group that contains from 1 to 8 carbon atoms and is optionally interrupted by an oxygen atom or represents a benzyl group, and
$R_2$ represents a hydrogen atom or an alkyl group containing from 1 to 4 carbon atoms, or
$R_1$ und $R_2$ together represent a trimethylene group or a tetramethylene group, and
$R_3$ represents a hydrogen atom, a fluorine atom, a chlorine atom, a hydroxy group or an acyloxy group having from 1 to 16 carbon atoms.

2. Corticoids of the general formula Ia according to claim 1,

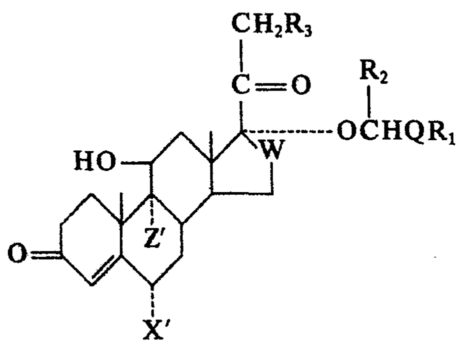

(Ia)

in which
W, Q, $R_1$, $R_2$ and $R_3$ have the meanings given in claim 1,
X′ represents a hydrogen atom, a fluorine atom or a methyl group, and
Z′ represents a hydrogen atom, a fluorine atom or a chlorine atom.

3. Corticoids of the general formula Ib according to claim 1

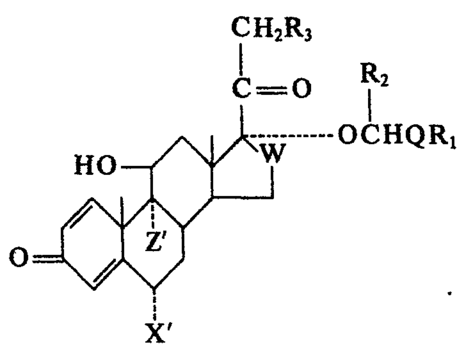

(Ib)

in which
X′, Z′, W, Q, $R_1$, $R_2$ und $R_3$ have the meanings given in claim 2.

4. Corticoids of the general formula Ic according to claim 1

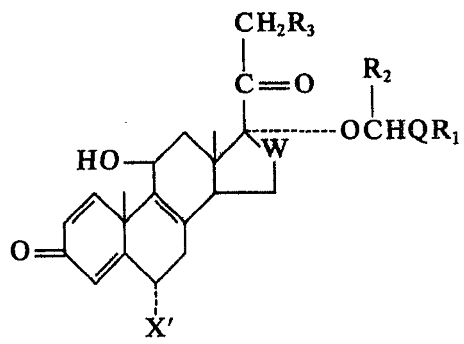

(Ic)

in which
X′, W, Q, $R_1$, $R_2$ and $R_3$ have the meanings given in claim 2.

5. Corticoids according to claims 2 to 4, characterised in that the symbol X′ of the general formulae represents a hydrogen atom.

6. Corticoids according to claims 2, 3 and 5, characterised in that the symbol Z′ of their general formulae represents a hydrogen atom.

7. Corticoids according to claims 2, 3 and 5, characterised in that the symbol Z′ of their general formulae represents a fluorine atom or a chlorine atom.

8. Corticoids according to claims 1 to 7, characterised in that the symbol W of their general formulae represents a methylene group.

9. Corticoids according to claims 1 to 8, characterised in that the symbol Q of their general formulae represents an oxygen atom.

10. Corticoids according to claims 1 to 8, characterised in that the symbol Q of their general formulae represents a sulphur atom.

11. Corticoids according to claims 1 to 10, characterised in that the symbol $R_2$ of their general formulae represents a hydrogen atom.

12. Corticoids according to claims 1 to 11, characterised in that the symbol $R_2$ of their general formulae represents an alkyl group containing from 1 to 6 carbon atoms.

13. Corticoids according to claims 1 to 12, characterised in that the symbol $R_3$ of their general formulae represents a hydroxy group or an acyloxy group containing from 1 to 8 carbon atoms.

14. Corticoids according to claims 1 to 12, characterised in that the symbol $R_3$ of their general formulae represents a fluorine atom or a chlorine atom.

15. Compound according to claim 1, namely 11β,21-dihydroxy-17α-methoxymethoxy-4-pregnene-3,20-dione.

16. Compound according to claim 1, namely 11β,21-dihydroxy-17α-(1′-methoxymethoxy)-4-pregnene-3,20-dione.

17. Compound according to claim 1, namely 17α-(1′-ethoxyethoxy)-11β,21-dihydroxy-4-pregnene-3,20-dione.

18. Compound according to claim 1, namely 11β,21-dihydroxy-17α-(1′-isobutoxyethoxy)-4-pregnene-3,20-dione.

19. Compound according to claim 1, namely 11β,21-dihydroxy-17α-(2′-tetrahydropyranyloxy)-4-pregnene-3,20-dione.

20. Compound according to claim 1, namely 11β,21-dihydroxy-17α-(1′-methoxyethoxy)-6α-methyl-4-pregnene-3,20-dione.

21. Compound according to claim 1, namely 11β,21-dihydroxy-17α-(1′-methoxyethoxy)-16β-methyl-4-pregnene-3,20-dione.

22. Compound according to claim 1, namely 11β-hydroxy-17α-(1′-methoxyethoxy)-4-pregnene-3,20-dione.

23. Compound according to claim 1, namely 11β,21-dihydroxy-17α-(2′-methoxyethoxymethoxy)-4-pregnene-3,20-dione.

24. Compound according to claim 1, namely 11β,21-dihydroxy-17α-methoxymethoxy-1,4-pregnadiene-3,20-dione.

25. Compound according to claim 1, namely 11β,21-dihydroxy-17α-(1′-methoxyethoxy)-6α-methyl-1,4-pregnadiene-3,20-dione.

26. Compound according to claim 1, namely 11β,21-dihydroxy-17α-ethoxymethoxy-4-pregnene-3,20-dione.

27. Compound according to claim 1, namely 11β,21-dihydroxy-17α-propoxymethoxy-4-pregnene-3,20-dione.

28. Compound according to claim 1, namely 11β,21-dihydroxy-17α-butoxymethoxy-4-pregnene-3,20-dione.

29. Compound according to claim 1, namely 11β,21-dihydroxy-6α-fluoro-17α-methoxymethoxy-4-pregnene-3,20-dione.

30. Compound according to claim 1, namely 11β,21-dihydroxy-17α-methoxymethoxy-16β-methyl-4-pregnene-3,20-dione.

31. Compound according to claim 1, namely 21-acetoxy-11β-hydroxy-17α-methoxymethoxy-4-pregnene-3,20-dione.

32. Compound according to claim 1, namely 11β-hydroxy-17α-methoxymethoxy-21-propionyloxy-4-pregnene-3,20-dione.

33. Compound according to claim 1, namely 21-butyryloxy-11β-hydroxy-11α-methoxymethoxy-4-pregnene-3,20-dione.

34. Compound according to claim 1, namely 11β-hydroxy-17α-methoxymethoxy-21-trimethylacetoxy-4-pregnene-3,20-dione.

35. Compound according to claim 1, namely 21-acetoxy-11β-hydroxy-17α-methoxymethoxy-1,4-pregnadiene-3,20-dione.

36. Compound according to claim 1, namely 21-butyryloxy-11β-17α-methoxymethoxy-1,4-pregnadiene-3,20-dione.

37. Compound according to claim 1, namely 21-acetoxy-17α-methoxymethoxy-4-pregnene-3,11,20-trione.

38. Compound according to claim 1, namely 21-acetoxy-9α-chloro-11β-hydroxy-17α-methoxymethoxy-4-pregnene-3,20-dione.

39. Compound according to claim 1, namely 21-acetoxy-9α-fluoro-11β-hydroxy-17α-methoxymethoxy-4-pregnene-3,20-dione.

40. Compound according to claim 1, namely 9α-chloro-11β,21-dihydroxy-17α-methoxymethoxy-4-pregnene-3,20-dione.

41. Compound according to claim 1, namely 21-acetoxy-11β-hydroxy-17α-methylthiomethoxy-1,4-pregnadiene-3,20-dione.

42. Compound according to claim 1, namely 21-acetoxy-9α-fluoro-11β-hydroxy-17α-methylthiomethoxy-4-pregnene-3,20-dione.

43. Compound according to claim 1, namely 21-acetoxy-9α-chloro-17α-(1,3,6-trioxaheptyl)-1,4-pregnadiene-3,20-dione.

44. Compound according to claim 1, namely 21-acetoxy-9α,11β-dichloro-17α-(1,3,6-trioxaheptyl)-1,4-pregnadiene-3,20-dione.

45. Compound according to claim 1, namely 21-acetoxy-9α-chloro-11β-hydroxy-17α-methoxymethoxy-1,4-pregnadiene-3,20-dione.

46. Compound according to claim 1, namely 21-acetoxy-9α,11β-dichloro-17α-methoxymethoxy-1,4-pregnadiene-3,20-dione.

47. Compound according to claim 1, namely 21-acetoxy-11β-hydroxy-17α-(1,3,6-trioxaheptyl)-1,4,8-pregnatriene-3,20-dione.

48. Compound according to claim 1, namely 9α-21-dichloro-11β-hydroxy-17α-methoxymethoxy-4-pregnene-3,20-dione.

49. Compound according to claim 1, namely 9α-chloro-21-fluoro-11β-hydroxy-17α-methoxymethoxy-1,4-pregnadiene-3,20-dione.

50. Compound according to claim 1, namely 9α-chloro-11β-hydroxy-17α-methoxymethoxy-1,4-pregnadiene-3,20-dione.

51. Compound according to claim 1, namely 21-fluoro-11β-hydroxy-17α-methoxymethoxy-1,4,8-pregnatriene-3,20-dione.

52. Compound according to claim 1, namely 21-chloro-11β-hydroxy-17α-methoxymethoxy-1,4,8-pregnatriene-3,20-dione.

53. Compound according to claim 1, namely 9α-chloro-21-fluoro-11β-hydroxy-17α-methoxymethoxy-4-pregnene-3,20-dione.

54. Compound according to claim 1, namely 9α-fluoro-11β-hydroxy-17α-methoxymethoxy-21-propionyloxy-1,4-pregnadiene-3,20-dione.

55. Compound according to claim 1, namely 21-butyryloxy-9α-fluoro-11β-hydroxy-17α-methoxymethoxy-1,4-pregnadiene-3,20-dione.

56. Compound according to claim 1, namely 21-butyryloxy-9α-fluoro-17α-methoxymethoxy-1,4-pregnadiene-3,11,20-trione.

57. Compound according to claim 1, namely 21-butyryloxy-9α-fluoro-17α-(1,3,6-trioxaheptyl)-1,4-pregnadiene-3,11,20-trione.

58. Compound according to claim 1, namely 9α-fluoro-11β-hydroxy-17α-methoxymethoxy-16β-methyl-21-propionyloxy-1,4-pregnadiene-3,20-dione.

59. Compound according to claim 1, namely 9α-fluoro-11β-hydroxy-17α-methoxymethoxy-16β-methyl-21-propionyloxy-4-pregnene-3,20-dione.

60. Compound according to claim 1, namely 9α-fluoro-11β-21-dihydroxy-17α-methoxymethoxy-16β-

methyl-1,4-pregnadiene-3,20-dione.

61. Compound according to claim 1, namely 21-chloro-9α-fluoro-11β-hydroxy-17α-methoxymethoxy-16β-methyl-1,4-pregnadiene-3,20-dione.

62. Compound according to claim 1, namely 21-butyryloxy-9α-fluoro-11β-hydroxy-17α-methoxymethoxy-4-pregnene-3,20-dione.

63. Compound according to claim 1, namely 9α-fluoro-11β-hydroxy-17α-methoxymethoxy-21-valeryloxy-4-pregnene-3,20-dione.

64. Compound according to claim 1, namely 21-chloro-9α-fluoro-11β-hydroxy-17α-methoxymethoxy-1,4-pregnadiene-3,20-dione.

65. Compound according to claim 1, namely 11β,21-dihydroxy-6-chloro-17α-methoxymethoxy-4,6-pregnadiene-3,20-dione.

66. Compound according to claim 1, namely 11β,21-dihydroxy-17α-isopropoxymethoxy-4-pregnene-3,20-dione.

67. Compound according to claim 1, namely 11β,21-dihydroxy-17α-isopropoxymethoxy-1,4-pregnadiene-3,20-dione.

68. Compound according to claim 1, namely 17α-ethoxymethoxy-11β,21-dihydroxy-1,4-pregnadiene-3,20-dione.

69. Compound according to claim 1, namely 11β-hydroxy-17α-methoxymethoxy-4-pregnene-3,20-dione.

70. Compound according to claim 1, namely 21-acetoxy-17α-ethoxymethoxy-9α-chloro-11β-hydroxy-4-pregnene-3,20-dione.

71. Compound according to claim 1, namely 11β,21-dihydroxy-17α-hexyloxymethoxy-4-pregnene-3,20-dione.

72. Compound according to claim 1, namely 17α-benzyloxymethoxy-11β,21-dihydroxy-4-pregnene-3,20-dione.

73. Compound according to claim 1, namely 11β,21-dihydroxy-17α-methoxymethoxy-16-methylene-4-pregnene-3,20-dione.

74. Compound according to claim 1, namely 11β,21-dihydroxy-17α-propoxymethoxy-1,4-pregnadiene-3,20-dione.

75. Compound according to claim 1, namely 21-acetoxy-11β-hydroxy-17α-methoxymethoxy-16-methylene-4-pregnene-3,20-dione.

76. Pharmaceutical preparations, characterised by a content of from 1 to 3 corticoids according to claims 1 to 75.

77. Process for the manufacture of corticoids of the general formula I according to claim 1, characterised in that, in a manner known per se, a 17α-hydroxysteroid of the general formula II

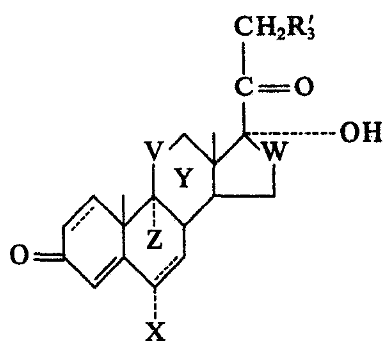

(II)

in which

$\underline{\cdots}$ , X, Y, Z, V and W have the meanings given in claim 1 and $R_3'$ represents a hydrogen atom, a fluorine atom, a chlorine atom or an esterified hydroxy group is reacted, optionally after intermediate protection of an 11β-hydroxy group,

a) with an acetal of the general formula III

$R_2HC(OR_1)_2$ (III)

in which
$R_1$ and $R_2$ have the meanings given in claim 1, or

b) with an α-haloether of the general formula IV

$Hal-R_2CH-OR_1$ (IV)

in which
$R_1$ and $R_2$ have the meanings given in claim 1 and Hal represents a chlorine atom, a bromine atom or an iodine atom, or

c) with a vinyl ether of the general formula V

$R_2'CH=CH-OR_1$ (V)

in which
$R_1$ has the meanings given in claim 1 and $R_2'$ represents a hydrogen atom or an alkyl radical containing from one to three carbon atoms, or

d) with a sulphoxide of the general formula VI

$R_2CH_2SOR_1$ (VI)

in which
$R_1$ and $R_2$ have the meanings given in claim 1, and, optionally, the corticoids obtained according to variants a) to d) that are saturated in the 1- and 2-position are dehydrogenated in the 1,2-position, and/or an 11$\beta$-hydroxy group is oxidised to form an oxo group, and/or 21-ester groups are hydrolysed or 21-hydroxy groups are esterified or replaced by fluorine atoms or chlorine atoms.

78. Process for the manufacture of corticoids of the general formula I according to claim 1, characterised in that, in a manner known per se,

a) chlorine or hypochlorous acid is added to a 9,11-dehydrosteroid of the general formula VII

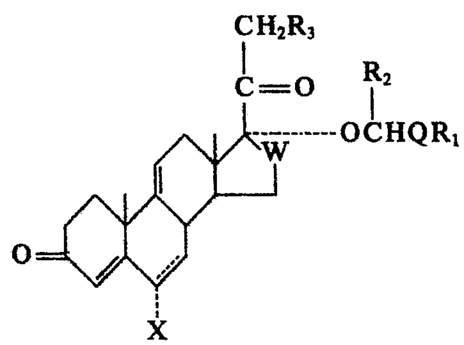

(VII)

in which
≡, X, W, Q, $R_1$, $R_2$ and $R_3$ have the meanings given in claim 1, or

b) the epoxy ring of a 9,11-epoxysteroid of the general formula VIII

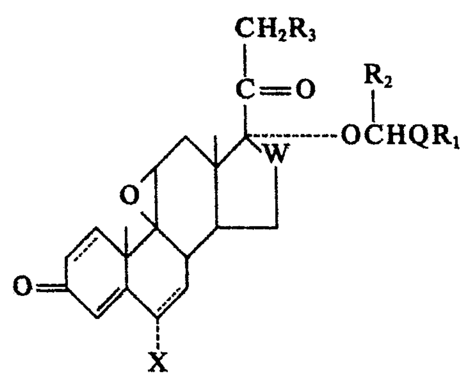

(VIII)

in which
≡, X, W, Q, $R_1$, $R_2$ and $R_3$ have the meanings given in claim 1 is opened with hydrogen fluoride or hydrogen chloride, or

c) Z″ or HZ″ ist split off from a 9-halosteroid of the general formula IX

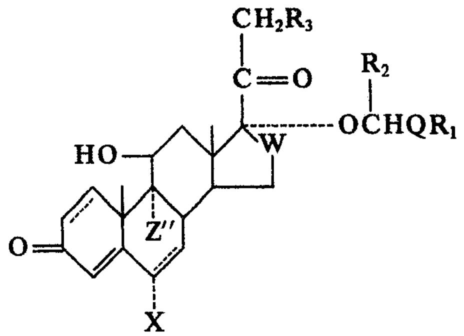

(IX)

in which
$\overset{\dots}{-}$ , X, W, Q, $R_1$, $R_2$ and $R_3$ have the meanings given in claim 1 and Z″ represents a chlorine atom or a bromine atom,
and, optionally, the corticoids obtained according to variants a) to c) that are saturated in the 1,2-position are dehydrogenated in the 1,2-position, and/or an 11$\beta$-hydroxy group is oxidised to form an oxo group, and/or 21-ester groups are hydrolysed or 21-hydroxy groups are esterified or replaced by fluorine atoms or chlorine atoms.

79. Process for the manufacture of 11$\beta$-hydroxysteroids of the general formula Id

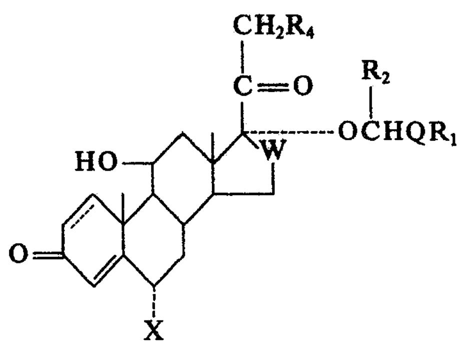

(Id)

in which
$\overset{\dots}{-}$ , X, W, Q, $R_1$ and $R_2$ have the meanings given in claim 1 and $R_4$ represents a hydrogen atom or a hydroxy group, characterised in that an 11-deoxysteroid of the general formula XI

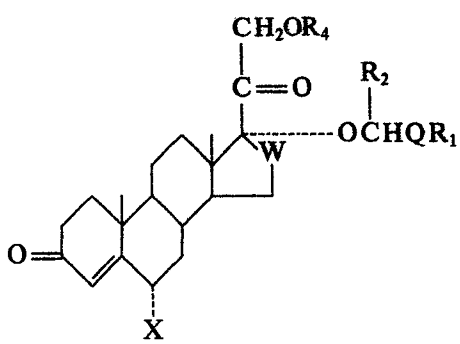

(XI)

in which
X, W, Q, $R_1$ and $R_2$ have the meanings given above and $R_4$ represents a hydrogen atom, a hydroxy group or an alkanoyloxy group containing from 1 to 6 carbon atoms is fermented with fungal cultures of the genus Curvularia and the resulting compounds of the formula Id in which $\overset{\dots}{-}$ represents a single bond are optionally dehydrogenated in the 1,2-position.

80. Process according to claim 79, characterised in that the fermentation is carried out using fungal cultures of the species Curvularia lunata.

**Revendications**

1. Corticostéroïdes répondant à la formule générale I:

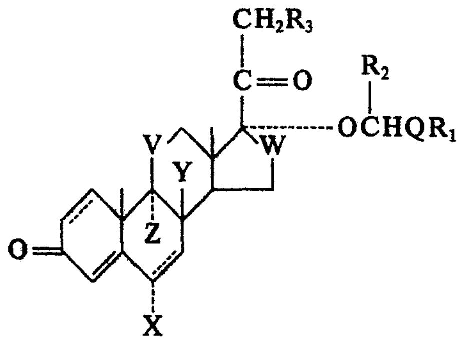

(I)

dans laquelle
les liaisons ---- sont des liaisons simples ou doubles,
X représente un atome d'hydrogène, de fluor ou de chlore ou un radical méthyle,
Y représente un atome d'hydrogène et
Z représente un atome d'hydrogène, de fluor ou de chlore,
ou encore
Y et Z forment ensemble une liaison carbone-carbone,
V représente un radical $\beta$-hydroxyméthylène, $\beta$-chlorométhylène ou carbonyle,
W représente un radical méthylène, éthylidène ou vinylidène,
Q représente un atome d'oxygène ou de soufre,
$R_1$ représente un radical alkyle contenant de 1 à 8 atomes de carbone, éventuellement interrompu par un atome d'oxygène, ou représente un radical benzyle, et
$R_2$ représente un atome d'hydrogène ou un radical alkyle contenant de l à 4 atomes de carbone,
ou encore
$R_1$ et $R_2$ forment ensemble un radical triméthylène ou tétraméthylène, et
$R_3$ représente un atome d'hydrogène, de fluor ou de chlore, un radical hydroxy ou un radical acyloxy contenant de 1 à 16 atomes de carbone.

2. Corticostéroïdes selon la revendication 1, qui répondent à la formule générale Ia:

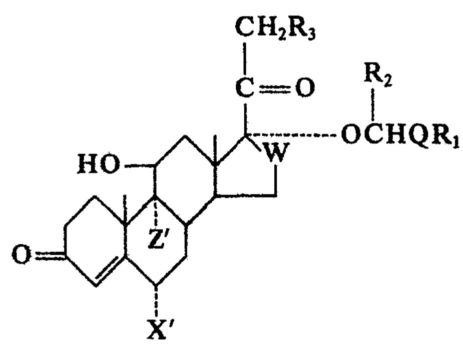

(Ia)

dans laquelle
W, Q, $R_1$, $R_2$ et $R_3$ ont les significations données à la revendication 1,
X' représente un atome d'hydrogène ou de fluor ou un radical méthyle et
Z' représente un atome d'hydrogéne, de fluor ou de chlore.

3. Corticostéroïdes selon la revendication 1, qui répondent à la formule générale Ib

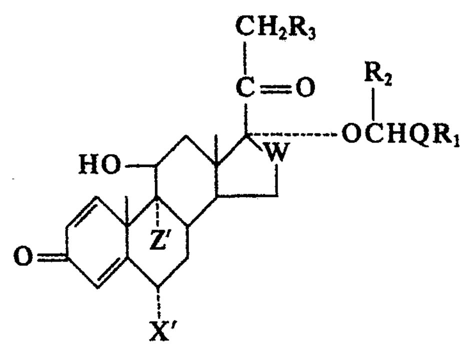

(Ib)

dans laquelle
X', Z', W, Q, $R_1$, $R_2$ et $R_3$ ont les significations données à la revendication 2.

4. Corticostéroïdes selon la revendication 1, qui répondent à la formule générale Ic

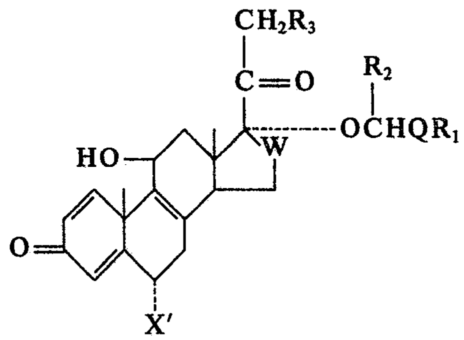

(Ic)

dans laquelle
X', W, Q, $R_1$, $R_2$ et $R_3$ ont les significations données à la revendication 2.

5. Corticostéroïdes selon l'une quelconque des revendications 2 à 4, caractérisés en ce que X' représente un atome d'hydrogène.

6. Corticostéroïdes l'une quelconque des revendications 2, 3 et 5, caractérisés en ce que Z' représente un atome d'hydrogène.

7. Corticostéroïdes selon l'une quelconque des revendications 2, 3 et 5, caractérisés en ce que Z' représente un atome de fluor ou de chlore.

8. Corticostéroïdes selon l'une quelconque des revendications 1 à 7, caractérisés en ce que W représente un radical méthylène.

9. Corticostéroïdes selon l'une quelconque des revendications 1 à 8, raractérisés en ce que Q représente un atome d'oxygène.

10. Corticostéroïdes selon l'une quelconque des revendications 1 à 8, caractérisés en ce que Q représente un atome de soufre.

11. Corticostéroïdes selon l'une quelconque des revendications 1 à 10, caractérisés en ce que $R_2$ représente un atome d'hydrogène.

12. Corticostéroïdes selon l'une quelconque des revendications 1 à 11, caractérisé en ce que $R_2$ représente un radical alkyle contenant de 1 à 6 atomes de carbone.

13. Corticostéroïdes selon l'une quelconque des revendications 1 à 12, caractérisés en ce que $R_3$ représente un radical hydroxy ou un radical acyloxy contenant de 1 à 8 atomes de carbone.

14. Corticostéroïdes selon l'une quelconque des revendications 1 à 12, caractérisés en ce que $R_3$ représente un atome de flour ou de chlore.

15. Composé selon la revendication 1, en l'espèce la dihydroxy-11$\beta$,21 méthoxyméthoxy-17$\alpha$ prégnéne-4 dione-3,20.

16. Composé selon la revendication 1, en l'espèce la dihydroxy-11$\beta$,21 (méthoxy-1 éthoxy)-17$\alpha$ prégnène-4 dione-3,20.

17. Composé selon la revendication 1, en l'espèce l'(éthoxy-1 éthoxy)-17$\alpha$ dihydroxy-11$\beta$,21 prégnène-4 dione-3,20.

18. Composé selon la revendication 1, en l'espèce la dihydroxy-11$\beta$,21(isobutyloxy-1 éthoxy)-17$\alpha$ prégnène-4 dione-3,20.

19. Composé selon la revendication 1, en l'espèce la dihydroxy-11$\beta$,21 (tétrahydropyrannyl-2-oxy)-17$\alpha$ prégnène-4-3,20.

20. Composé selon la revendication 1, en l'espèce la dihydroxy-11$\beta$,21 (méthoxy-1 éthoxy)-17$\alpha$ méthyl-6$\alpha$ prégnène-4 dione-3,20.

21. Composé selon la revendication 1, en l'espèce la dihydroxy-11$\beta$,21 (méthoxy-1 éthoxy)-17$\alpha$ méthyl-16$\beta$ prégnène-4 dione-3,20.

22. Composé selon la revendication 1, en l'espèce l'hydroxy-11$\beta$ (méthoxy-1 éthoxy)-17$\alpha$ prégnène-4 dione-3,20.

23. Composé selon la revendication 1, en l'espèce la dihydroxy-11$\beta$,21 (méthoxy-2 éthoxyméthoxy)-17$\alpha$ prégnène-4 dione-3,20.

24. Composé selon la revendication 1, en l'espèce la dihydroxy-11$\beta$,21 méthoxyméthoxy-17$\alpha$ prégnadiène-1,4 dione-3,20.

25. Composé selon la revendication 1, en l'espèce la dihydroxy-11$\beta$,21 (méthoxy-1 éthoxy)-17$\alpha$ méthyl-6$\alpha$ prégnadiène-1,4 dione-3,20.

26. Composé selon la revendication 1, en l'espèce la dihydroxy-11$\beta$,21 éthoxyméthoxy-17$\alpha$ prégnène-4 dione-3,20.

27. Composé selon la revendication 1, en l'espèce la dihydroxy-11$\beta$,21 propoxyméthoxy-17$\alpha$ pré-

gnène-4 dione-3,20.

28. Composé selon la revendication 1, en l'espèce la dihydroxy-11β,21 butoxyméthoxy-17α prégnène-4 dione-3,20.

29. Composé selon la revendication 1, en l'espèce la dihydroxy-11β,21 fluoro-6α méthoxyméthoxy-17α prégnène-4 dione-3,20.

30. Composé selon la revendication 1, en l'espèce la dihydroxy-11β,21 méthoxyméthoxy-17α méthyl-16β prégnène-4-dione-3,20.

31. Composé selon la revendication 1, en l'espèce l'acétoxy-21 hydroxy-11β méthoxyméthoxy-17α prégnène-4 dione-3,20.

32. Composé selon la revendication 1, en l'espèce l'hydroxy-11β méthoxyméthoxy-17α propionyloxy-21 prégnène-4 dione-3,20.

33. Composé selon la revendication 1, en l'espèce la butyryloxy-21 hydroxy-11β méthoxyméthoxy-17α prégnène-4 dione-3,20.

34. Composé selon la revendication 1, en l'espèce l'hydroxy-11β méthoxyméthoxy-17α triméthylacétoxy-21 prégnène-4 dione-3,20.

35. Composé selon la revendication 1, en l'espèce l'acétoxy-21 hydroxy-11β méthoxyméthoxy-17α prégnadiène-1,4 dione-3,20.

36. Composé selon la revendication 1, en l'espèce la butyryloxy-21 hydroxy-11β méthoxyméthoxy--17α prégnadiène-1,4 dione-3,20.

37. Composé selon la revendication 1, en l'espèce l'acétoxy-21 méthoxyméthoxy-17α prégnène-4 trione-3,11,20.

38. Composé selon la revendication 1, en l'espèce l'acétoxy-21 chloro-9α hydroxy-11β méthoxyméthoxy-17α prégnè-4 dione-3,20.

39. Composé selon la revendication 1, en l'espèce l'acétoxy-21 fluoro-9α hydroxy-11β méthoxyméthoxy-17α prégnène-4 dione-3,20.

40. Composé selon la revendication 1, en l'espèce la chloro-9α dihydroxy-11β,21-méthoxyméthoxy--17α prégnène-4 dione-3,20.

41. Composé selon la revendication 1, en l'espèce l'acétoxy-21 hydroxy-11β méthylthiométhoxy-17α prégnadiène-1,4 dione-3,20.

42. Composé selon la revendication 1, en l'espèce l'acétoxy-21 fluoro-9α hydroxy-11β méthylthiométhoxy-17α prégnène-4 dione-3,20.

43. Composé selon la revendication 1, en l'espèce l'acétoxy-21 chloro-9α (trioxa-1,3,6 heptyl)-17α prégnadiène-1,4 dione-3,20.

44. Composé selon la revendication 1, en l'espèce l'acétoxy-21 dichloro-9α,11β (trioxa-1,3,6 heptyl)-17α prégnadiène-1,4 dione-3,20.

45. Composé selon la revendication 1, en l'espèce l'acétoxy-21 chloro-9α hydroxy-11β méthoxyméthoxy-17α prégnadiène dione-3,20.

46. Composé selon la revendication 1, en l'espèce l'acétoxy-21 dichloro-9α,11β méthoxyméthoxy-17α prégnadiène-1,4 dione-3,20.

47. Composé selon la revendication 1, en l'espèce l'acétoxy-21 hydroxy-11β (trioxa-1,3,6 heptyl)-17α prégnatriène-1,4,8 dione-3,20.

48. Composé selon la revendication 1, en l'espèce la dichloro-9α,21 hydroxy-11β méthoxyméthoxy-17α prégnène-4 dione-3,20.

49. Composé selon la revendication 1, en l'espèce la chloro-9α fluoro-21 hydroxy-11β méthoxyméthoxy-17α prégnadiène-1,4 dione-3,20.

50. Composé selon la revendication 1, en l'espèce la chloro-9α hydroxy-11β méthoxyméthoxy-17α prégnadiène-1,4 dione-3,20.

51. Composé selon la revendication 1, en l'espèce la fluoro-21 hydroxy-11β méthoxyméthoxy-17α prégnatriène-1,4,8 dione-3,20.

52. Composé selon la revendication 1, en l'espèce la chloro-21 hydroxy-11β méthoxyméthoxy-17α prégnatriène-1,4,8 dione-3,20.

53. Composé selon la revendication 1, en l'espèce la chloro-9α fluoro-21 hydroxy-11β méthoxyméthoxy-17α prégnéne-4 dione-3,20.

54. Composé selon la revendication 1, en l'espèce la fluoro-9α hydroxy-11β méthoxyméthoxy-17α propionyloxy-21 prégnadiène-1,4 dione-3,20.

55. Composé selon la revendication 1, en l'espèce la butyryloxy-21 fluoro-9α hydroxy-11β méthoxymethoxy-17α prégnadiène-1,4 dione-3,20.

56. Composé selon la revendication 1, en l'espèce la butyryloxy-21 fluoro-9α méthoxyméthoxy-17α prégnadiène-1,4 trione-3,11,20.

57. Composé selon la revendication 1, en l'espèce la butyryloxy-21 fluoro-9α (trioxa-1,3,6 heptyl)-17α prégnadiène-1,4 trione-3,11,20.

58. Composé selon la revendication 1, en l'espèce la fluoro-9α hydroxy-11β méthoxyméthoxy-17α méthyl-16β propionyloxy-21 prégnadiène-1,4 dione-3,20.

59. Composé selon la revendication 1, en l'espèce la fluoro-9α hydroxy-11β méthoxyméthoxy-17α méthyl-16β propionyloxy-21 prégnène-4 dione-3,20.

60. Composé selon la revendication 1, en l'espèce la fluoro-9α dihydroxy-11β,21 méthoxyméthoxy-17α méthyl-16β prégnadiène-1,4 dione-3,20.

61. Composé selon la revendication 1, en l'espèce la chloro-21 fluoro-9α hydroxy-11β méthoxyméthoxy-17α méthyl-16β prégnadiène-1,4 dione-3,20.

62. Composé selon la revendication 1, en l'espèce butyryloxy-21 fluoro-9α hydroxy-11β méthoxyméthoxy-17α prégnène-4 dione-3,20.

63. Composé selon la revendication 1, en l'espèce la fluoro-9α hydroxy-11β méthoxyméthoxy-17α pentanoyloxy-21 prégnène-4 dione-3,20.

64. Composé selon la revendication 1, en l'espèce la chloro-21 fluoro-9α hydroxy-11β méthoxyméthoxy-17α prégnadiène-1,4 dione-3,20.

65. Composé selon la revendication 1, en l'espèce la dihydroxy-11β,21 chloro-6 méthoxyméthoxy-17α prégnadiène-4,6 dione-3,20.

66. Composé selon la revendication 1, en l'espèce la dihydroxy-11β,21 isopropoxyméthoxy-17α prégnène-4 dione-3,20.

67. Composé selon la revendication 1, en l'espèce la dihydroxy-11β,21 isopropoxyméthoxy-17α prégnadiene-1,4 dione-3,20.

68. Composé selon la revendication 1, en l'espèce l'éthoxyméthoxy-17α dihydroxy-11β,21 prégnadiène-1,4 dione-3,20.

69. Composé selon la revendication 1, en l'espèce l'hydroxy-11β méthoxyméthoxy-17α prégnène-4 dione-20.

70. Composé selon la revendication 1, en l'espèce l'acétoxy-21 éthoxyméthoxy-17α chloro-9α hydroxy-11β prégnène-4 dione-3,20.

71. Composé selon la revendication 1, en l'espèce la dihydroxy-11β,21 hexyloxyméthoxy-17α prégnè-4 dione-3,20.

72. Composé selon la revendication 1, en l'espèce la benzyloxyméthoxy-17α dihydroxy-11β,21 prégnène-4 dione-3,20.

73. Composé selon la revendication 1, en l'espèce la dihydroxy-11β,21 méthoxyméthoxy-17α méthylène-16 prégnène-4 dione-3,20.

74. Composé selon la revendication 1, en l'espèce la dihydroxy-11β,21 propoxyméthoxy-17α prégnadiène-1,4 dione-3,20.

75. Composé selon la revendication 1, en l'espèce l'acétoxy-21 hydroxy-11β méthoxyméthoxy-17α méthylène-16 prégnène-4 dione-3,20.

76. Médicaments caractérisés en ce qu'ils contiennent de 1à 3 corticostéroïdes selon l'une quelconque des revendications 1 à 75.

77. Procédé de préparation de corticostéroïdes de formule générale I selon la revendication 1, procédé caractérisé en ce que, en opérant de manière connue, on fait réagir un hydroxy-17α stéroïde répondant à la formule générale II:

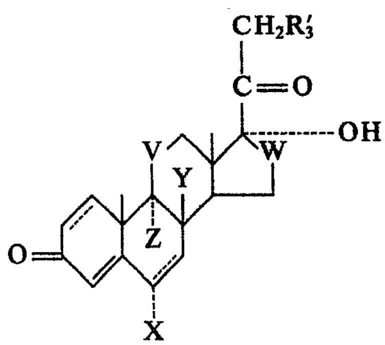

(II)

dans laquelle

====, X, Y, Z, V et W ont les significations données à la revendication 1 et $R_3'$ représente un atome d'hydrogène, de fluor ou de chlore ou un radical hydroxy estérifié, éventuellement après avoir protégé intermédiairement un radical hydroxy en 11β:

a) avec un acétal répondant à la formule générale III

$$R_2HC(OR_1)_2 \qquad (III)$$

dans laquelle $R_1$ et $R_2$ ont les significations données à la revendication 1, ou

b) avec un éther halogéné en α qui répond à la formule générale IV:

$$Hal-R_2CH-OR_1 \qquad (IV)$$

dans laquelle $R_1$ et $R_2$ ont les significations données à la revendication 1 et Hal désigne un atome de chlore, de brome ou d'iode, ou

c) avec un éther vinylique répondant à la formule générale V:

$R_2'CH=CH-OR_1$ (V)

dans laquelle $R_1$ à la signification donnée à la revendication 1 et $R_2'$ représente un atome d'hydrogène ou un radical alkyle contenant de 1 à 3 atomes de carbone, ou

d) avec un sulfoxyde répondant à la formule générale VI:

$R_2CH_2SOR_1$ (VI)

dans laquelle $R_1$ et $R_2$ ont les significations données à la revendication 1, et éventuellement on déshydrogène en 1,2 les corticostéroïdes obtenus qui sont saturés en cette position et/ou on oxyde un groupe hydroxy en 11$\beta$ en un groupe oxo et/ou on saponifie un groupe ester en 21 et/ou on estérifie un groupe hydroxy en 21 ou on l'échange contre un atome de fluor ou de chlore.

78. Procédé de préparation de corticostéroïdes selon la revendication 1, procédé caractérisé en ce que, en opérant de manière counne:

a) on fixe du chlore ou de l'acide hypochloreux sur un déhydro-9,11 stéroïde répondant à la formule générale VII:

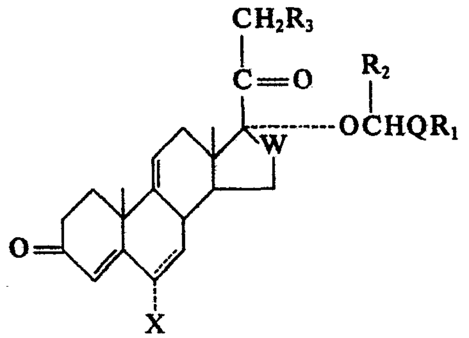
(VII)

dans laquelle

$\underline{\underline{\cdots}}$, X, W, Q, $R_1$, $R_2$ et $R_3$ ont les significations données à la revendication 1, ou

b) on ouvre le noyau époxy d'un époxy-9,11 stéroïde répondant à la formule générale VIII:

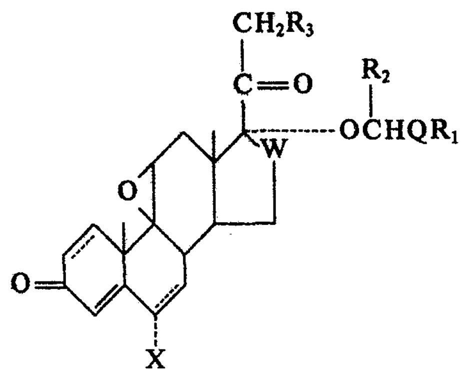
(VIII)

dans laquelle

$\underline{\underline{\cdots}}$, X, W, Q, $R_1$, $R_2$ et $R_3$ ont les significations données à la revendication 1, par du fluorure d'hydrogène ou du chlorure d'hydrogène, ou

c) on enlève Z″ ou HZ″ d'un halogéno-9 stéroïde répondant à la formule générale IX:

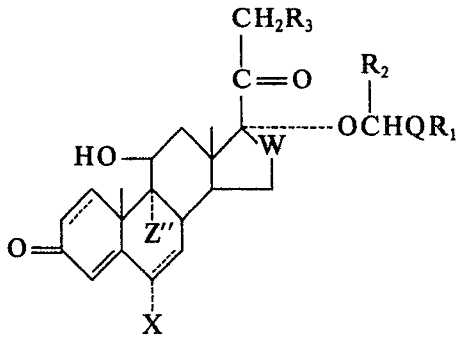

(IX)

dans laquelle
==== , X, W, Q, $R_1$, $R_2$ et $R_3$ ont les significations données à la revendication 1 et Z″ représente un atome de chlore ou de brome,
et éventuellement on déshydrogène en 1,2 les corticostéroïdes obtenus selon a) à c) qui sont saturés en cette position et/ou on oxyde un groupe hydroxy en 11$\beta$ en un groupe oxo et/ou on saponifie un groupe ester en 21 ou on estérifie un groupe hydroxy en 21 ou on l'échange contre un atome de fluor ou de chlore.

79. Procédé de préparation d'hydroxy-11$\beta$ stéroïdes répondant à la formule générale Id:

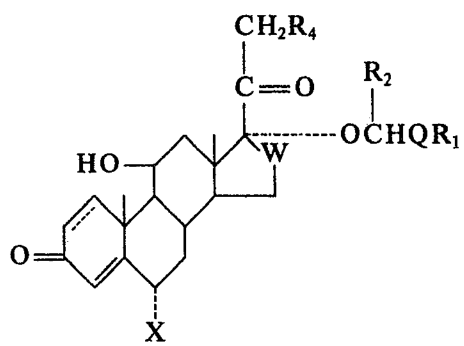

(Id)

dans laquelle
==== , X, W, Q, $R_1$ et $R_2$ ont les significations données à la revendication 1 et $R_4$ désigne un atome d'hydrogène ou un radical hydroxy, procédé caractérisé en ce qu'on fait fermenter un désoxy-11 stéroïde répondant à la formule générale XI:

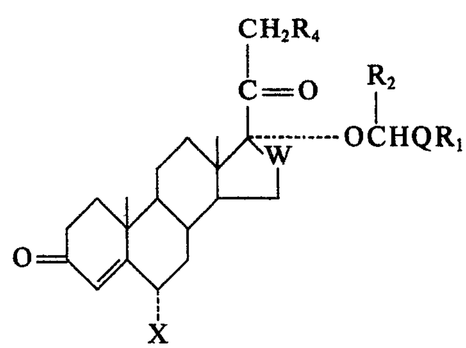

(XI)

dans laquelle
X, W, Q, $R_1$ et $R_2$ ont les significations indiquées ci-dessus et $R_4$ représente un atome d'hydrogène, un radical hydroxy ou un radical alcanoyloxy contenant de 1 à 6 atomes de carbone, au moyen d'une culture d'un mycète du genre Curvularia, et on déshydrogène éventuellement en position 1,2 les composés de formule Id obtenus pour lesquels ==== désigne une liaison simple.

80. Procédé selon la revendication 79, caractérisé en ce qu'on effectue la fermentation au moyen d'une culture d'un mycète de l'espèce Curvularia lunata.